# EUROPEAN PATENT APPLICATION

(11) **EP 4 707 804 A2**
(43) Date of publication of application: **11.03.2026**
(21) Application number: 25224842.2
(22) Date of filing: 18.12.2024
(51) Int. Cl.: G01N 33/53

(54) **LECTIN-BASED ASSAY FOR DETECTING MICROBIAL CONTAMINATION**

(30) Priority: 19.12.2023 EP 23218073
(62) Divisional of application: 24820925.6
(71) Applicant: The Antibody Lab GmbH, 1210 Wien (AT)
(72) Inventor: HIMMLER, Gottfried, 2301 Groß-Enzersdorf (AT); WOLOSZCZUK, Wolfgang, 1040 Wien (AT); MARENZI, Luca, 1030 Wien (AT); TESARZ, Manfred, 1210 Wien (AT)
(74) Representative: Redl, Gerda

(57) **Abstract**

An analytical method to detect microbial contaminants in an oily liquid, comprising:
a) preparing an aqueous liquid from the oily liquid, thereby obtaining glycan-containing microbial material originating from any microbial contaminants comprised in the oily liquid;
b) determining the presence of glycans in the aqueous liquid using a lectin-based lateral-flow assay (LFA) that employs mannose-binding protein (MBP) as capture agent of a test region of a lateral flow membrane and as capture agent bound to flowable, identifiable reporter nanoparticles, using a capture reaction buffer (CRB) which comprises 100 to 500 mM free calcium ions, a detergent, a buffer agent and a pH ranging between 6.5 and 9.0, thereby allowing identification of an MBP sandwiched capture product at the test region, which indicates microbial contamination; and
a test kit for use in the method, which comprises:
a) a lectin-based lateral-flow assay device, which employs mannose-binding protein (MBP) as capture agent that is bound to flowable, identifiable reporter nanoparticles at a reaction zone of a lateral flow membrane; and
b) a running buffer which comprises a detergent, a buffer agent and a pH ranging between 6.5 and 9.0, which running buffer comprises 100 to 500 mM free calcium ions and sets an amount of 100 to 500 mM free calcium ions at the reaction zone,
wherein the reaction zone comprises the MBP bound to the reporter nanoparticles at a conjugate zone, and a test region comprising a test line where immobilized MBP is used as a capture agent.

## Description

### FIELD OF THE INVENTION

The invention refers to a method of determining microbial contamination in oily liquids, such as oils, in particular crude oil, petroleum or fractions thereof. The method makes use of a lateral-flow assay (LFA) that employs a mannose-binding protein (MBP) as a capture agent to react with glycans of contaminating microbes, in particular mannose or mannan. By using a specific reaction buffer, the sensitivity of the LFA can be improved despite of hydrophobic substances in a sample that is tested with the LFA, which hydrophobic substances can interfere with the MBP-glycan- or MBP-mannose-reaction.

### BACKGROUND OF THE INVENTION

Detection of microbial contamination in non-aqueous fluids such as diesel and kerosene is notoriously difficult as many different organisms are able to grow either in these liquids or in the interface between hydrophobic and aqueous phases. A test for such microbial growth may be used as an indicator of problems with the storage or use of such liquids.

There are a number of different approaches available to test for microbial contamination, such as growth tests on solid or in liquid substrates, the detection of ATP as a surrogate of metabolic activities, nucleic acid-based tests and tests for microbial antigens of target microorganisms by specific antibodies. The most simple and rapid form of assay for such purposes are lateral flow assays as they can be performed on site and require relatively little equipment and know-how.

Lateral flow immunoassays (LFIAs) have been used to test microbial contamination in fuel tanks using antibodies targeting antigens of microbial structures. ASTM International has adopted a standard test method, ASTM D8070, for screening fuels and fuel-associated aqueous specimens for microbial contamination by LFIAs. This test method is semi-quantitative and can be used to determine whether contamination in samples drawn from fuel tanks and systems is present within specific defined ranges. The test method measures the presence of microbial and metabolite antigens in a specimen, which are generated from the living cells and metabolites created by certain fungi and certain bacteria during growth on fuel. The presence of antigens is an indicator of microbial contamination in fuel systems. The test method is intended to provide a tool for assessing whether fuel storage and distribution facilities, or end-user fuel tanks, are subject to microbial growth, and to alert fuel suppliers or users to the potential for fuel quality or operational problems or the requirement for preventative or remedial measures, or both.

The disadvantages of the ASTM D8070 is its limited sensitivity: The ASTM D8070 test method may not be as sensitive as other methods, which could potentially lead to false-negative results. In addition, this LFIA is an antibody-based test for detecting certain microbes or microbial matter that frequently occur in fuel tanks. Thus, although the analytical sensitivity for some the target molecules of the antibodies utilized in such an assay may be acceptably high, the test may not be able to detect all microbes and microbial matter for its restricted specificity. The topic of relative focused specificity can be addressed by either choosing for the test antibodies that target generic microbial targets (widely shared antigens) or increase the number of targets (and thus consequently the number of antibody specificities in the assay).

It has been proposed to target shared antigens by the use lectins and Toll-like-Receptors as ligands in such assays to detect microbial matter (e.g. WO2021252902A1, EP3081937B1 and US9593160B2) with broad specificity and therefore high practical sensitivity. This is an approach well-known for reaction in aqueous matrices, as lectins and TLRs have been selected in nature to work in such matrices.

However, the specificity and affinity of lectins can be influenced by traces of hydrophobic substances in the assay buffer. The presence of hydrophobic substances in the assay buffer may affect the conformation of glycoproteins or the accessibility of specific glycan structures, thereby influencing the binding affinity of lectins. Additionally, hydrophobic substances can potentially interfere with the binding sites of lectins, leading to changes in their specificity and affinity for glycan motifs.

In order to perform an assay which is able to detect most bacterial and fungal matter (living or dead) with a high sensitivity in fuels such as kerosene or diesel and oils or greases are typically extracted with an aqueous extraction buffer before starting the LFA procedure, however, extracts may still comprise hydrophobic remainders. Fading of the signal line over time in lateral flow immunoassays can present challenges, particularly regarding sensitivity, robustness, accuracy and reliability of test results. Standard and well-known measures to counter fading of signals in LFAs are the use of appropriate nanoparticles, the use of covalent linkage chemistry, the use of optimized membranes, the removal of interfering substances, the control of flow rates and appropriate reading time.

A prominent generic target on microbes and microbial products is the sugar motif mannose, either as part of high mannose glycoprotein structures or as polymannan or as part of the cell walls. As a broad specificity lectin, a mannose-binding protein (MBP), also known as mannan-binding protein, is therefore a candidate binding molecule to detect microbial structures in samples. MBPs are known to recognize mannose-containing structures, such as those present on the surface of bacteria, fungi, and viruses. Some MBPs can also recognize N-acetylglucosamine, a sugar molecule present on the surface of some bacteria and fungi, they can also bind to other sugar molecules, such as fucose and glucose, as well as to lipopolysaccharides (LPS), a component of the outer membrane of Gram-negative bacteria.

MBPs are C-type lectins which are calcium-dependent carbohydrate-binding proteins usually forming oligomeric structures for efficient function. Natural MBP polypeptide chains may consist of various regions e.g., a collagen like region, an alpha-helical coiled-coil, and a C-type lectin domain (CTLD). While the collagen-like region and the coiled-coil region facilitate multimerization, the C-type-lectin domain provides the carbohydrate specificity of the MBPs. The C-type lectin fold is known to be a compact domain of 110-130 amino acid residues with a double-looped, two-stranded antiparallel β-sheet formed by the amino-and carboxy-terminal residues connected by two α-helices and a three-stranded antiparallel β-sheet. Calcium binding in C-type lectins is mediated by specific amino acid residues that coordinate calcium and bind the hydroxyl groups of sugars. This CTLD has two highly conserved disulfide bonds and up to four sites for binding Ca⁺⁺, with site occupancy depending on the lectin. Amino acid residues with carbonyl side chains are often coordinated to Ca⁺⁺ in the carbohydrate recognition domain (CRD), and these residues directly bind to sugars when Ca⁺⁺ is bound. A ternary complex can be formed between a sugar in a glycan, the Ca⁺⁺ ion, and amino acids within the CRD. Changes in amino acids within the CRD may alter sugar specificity. Key conserved residues that bind mannose and Ca⁺⁺ often include the "EPN" and "WND" amino acid motifs within the CRD of C-type lectins.

The presence of co-factors, such as divalent cations other than calcium, can influence MBL binding activity. Usually, calcium concentrations in the range of 1-10 mM are commonly employed to facilitate the recognition of mannose-containing structures by MBPs.US10696733B2 discloses microparticles for diagnosing an infection, which microparticles are coated with a mannose binding lectin, and its reaction with a mannose structure in a sample. After addition of a processing buffer, the sample typically comprises 5 mM Calcium besides a detergent, a DNAse, plasmin and salts.

WO2013012924A2 discloses engineered microbe-targeting or microbe-binding molecules to capture a microbe. The microbe targeting molecule can comprise a CRD of a C-type lectin such as human mannose-binding lectin. After addition of a processing buffer, a sample comprises 5 mM Calcium besides detergent(s), DNase, plasmin, and salts.

WO2021252902 discloses an LFA using microbe-targeting molecules comprising e.g., human mannose-binding lectin. Suitable running buffers are described to contain 10 mM CaCl₂, besides detergent(s), glucose and salts.

WO02/068959A2 discloses a detection method of analysis of a hydrocarbon fuel for the presence of a micro-organism using an antibody reactive with the micro-organism. WO2010102285A1 discloses a lateral flow glycan-detecting device for detecting a particular type of glycan in a bodily fluid using a conjugate containing a first lectin and a label, and an immobilized second lectin.

US2019077850A1 discloses microbe-binding molecules comprising a microbe surface-binding domain linked to a portion of an Fc region, for example mannose-binding lectin bound to an antibody Fc (FcMBL). Protein A-expressing and protein G- expressing microbes can bind to microbe-binding molecules via two independent (but additive) mechanisms: Fc-mediated binding and microbe surface-binding domain A chelating agent can be used to remove free calcium ions for binding protein A- or protein G-expressing microbes via the Fc part of the molecule.

WO2019064463A1 disclose a method for concentrating a target virus or bacterium by capturing via a metal ion-dependent ligand-binding molecule and separating from the ligand-binding molecule by treatment with a chelating agent.

US2021364491A1 discloses a method of detecting biocontamination in a fuel sample using a specific biorecognition element.

DE102014116204B3 discloses detecting microbial contamination in an organic liquid by qPCR.

Li Zezhen et al. (American Journal of Biochemistry and Biotechnology 2018, 14(2):117-123) describe detection of sulfate-reducing bacteria in jet fuel by loop-mediated isothermal amplification combined with a lateral flow dipstick.

Climent Estela et al. (ACS Sensors 2020, 6(1):2379-3694) describe detecting microbial contamination of fuels by determining microbial genomic DNA in fuel extracts.

There is a need for a highly sensitive and robust assay for determining a broad spectrum of microbial contaminants in hydrophobic fluids, such as oily liquids, in particular in fuels.

### SUMMARY OF THE INVENTION

This summary is provided to introduce a selection of concepts in a simplified form that are further described below in the detailed description. This summary is not intended to identify key or essential features of the claimed subject matter, nor is it intended to be used to limit the scope of the claimed subject matter. Other features, details, utilities, and advantages of the claimed subject matter will be apparent from the following written detailed description, including those aspects illustrated in the accompanying drawings and defined in the appended claims.

It is the objective of the invention to provide a new strategy or method of determining, detecting and/or controlling microbial contamination in hydrophobic fluids such as oily liquids, in particular in fuels. It is a particular objective to ensure the quality of any such hydrophobic fluids by determining the absence or presence of microbial contaminants with a sensitive assay. It is a particular objective to provide an improved LFA using a MBP as capture agent.

The objective is solved by the claimed subject matter, and as further described herein.

The invention provides for an analytical method to detect microbial contaminants in an oily liquid, in particular a sample of an oily liquid, comprising:
a) preparing an aqueous liquid from the oily liquid, thereby obtaining glycan-containing microbial material originating from any microbial contaminants comprised in the oily liquid;
b) determining the presence of glycans in the aqueous liquid using a lectin-based lateral-flow assay (LFA) that employs a mannose-binding protein (MBP) as capture agent of a test region of a lateral flow membrane and as capture agent bound to flowable, identifiable reporter nanoparticles, using a capture reaction buffer (CRB) which comprises 100 to 500 mM free calcium ions, a detergent, a buffer agent and a pH ranging between 6.5 and 9, thereby allowing identification of an MBP sandwiched capture product at the test region, which indicates microbial contamination.

The invention provides for a surprisingly sensitive and robust LFA under certain assay conditions by using a MBP with aqueous extracts or fractions of an oily liquid. Hydrophobic or oily remainders in such aqueous extracts or fractions were found to not interfere with the assay's high sensitivity and robustness.

It was found that MBP capturing its target structure in the presence of a CRB as described herein, greatly improves the LFA.

The target structure of MBP (herein also called the target analyte) is herein understood as the structure or compound which is recognized and bound by MBP to detect an analyte comprising such structure. It is typically a glycan structure, in particular a mannan or mannose containing structure.

It surprisingly turned out that high concentrations of free calcium ions present provide for an increase of MBP binding to its target structure, in terms of sensitivity and stability of binding. In particular, a robust and sensitive LFA was provided with an amount of free calcium ions ranging from 100 to 500 mM to support MBP binding to the target structure.

Amounts of free calcium ions can be present in a buffer system, which provides for the amount of free calcium ions ranging from 100 to 500 mM in the CRB, thereby providing for the capture reaction at high sensitivity and without interference by remainders of the oily liquid in the aqueous liquid that was prepared from the oily liquid.

Besides the amount of free calcium ions, the CRB comprises advantageously a detergent, a buffer agent and a pH ranging between 6.5 and 9.0, preferably a pH of about (i.e., +/- 0.5 or +/- 0.25) 7 or 8.

Specifically, a buffer system is conveniently used in the LFA, which comprises one or more liquids or buffers. For example, an extraction liquid or buffer can be used to provide an extract of the oily liquid. The extract may be used with or without a running buffer. If the extract is directly applied onto the LFA device, in particular at a sample zone of the lateral flow membrane, the extract medium for the lateral flow within the LFA device, and the LFA can be carried out without a separate running buffer. The extract medium can e.g., be an extraction buffer that is used as a running buffer. In other words, a running buffer can also be used as an extraction buffer. Still, a separate running buffer can be used in addition to an extraction buffer. Additionally, a chase buffer can be used.

Typically, a buffer can be comprised at a specific area of the LFA device, in particular at or on a lateral flow membrane, preferably in a dry form e.g., dried onto the specific area. The reaction zone of the LFA device may contain a medium that is usually a buffer which is comprised at a specific area within the reaction zone. Specifically, a conjugate buffer is comprised at a conjugate zone, and/or a detection buffer is comprised at a test region and/or at a control region. In addition, a sample buffer can be used which is typically comprised at a sample zone of the LFA device, such as a sample pad at or on a lateral flow membrane.

Specifically, cumulative amounts of free calcium ions are originating from the buffer system such that the total amount of 100 to 500 mM free calcium ions is present during the MBP binding reactions to the MBP target structures.

Specifically, a buffer system is used which sets the composition of the CRB at the reaction zone of an LFA device, in particular of the lateral flow membrane of the LFA device, thereby providing the required amount of free calcium ions, 100 to 500 mM, in the zone where MBP is binding (i.e., capturing) its target structure.

The reaction zone of an LFA device in particular of the lateral flow membrane of the LFA device, specifically comprises
(i) a conjugate zone (also referred to as conjugate pad) comprising the MBP that is bound to the reporter nanoparticles (also referred to as MBP reporter nanoparticles); and
(ii) a detection zone which comprises the test region, and optionally a control region.

The test region typically comprises or consists of a test line or spot or area of the test region where MBP is immobilized.

The control region typically comprises or consists of a control line or spot or area of the control region where a control is immobilized.

Specifically, the method described herein provides for the composition of the CRB described herein:
a) at the conjugate zone of a lateral flow membrane;
b) at the reaction zone of the lateral flow membrane, in particular at a test region of the lateral flow membrane, in particular at the test region within the detection zone, and optionally at the control region within the detection zone.

Specifically, a buffer system is used to set a total amount of 100 to 500 mM free calcium ions in the CRB, thereby allowing a high amount of free calcium ions when MBP is capturing the target structure.

Specifically, a running buffer is used which comprises 100 to 500 mM free calcium ions, or an amount of free calcium ions which complements an amount of free calcium ions that is provided in the aqueous liquid (e.g., an aqueous extract) and/or within a device used for the LFA, to set a total amount of 100 to 500 mM free calcium ions in the CRB, thereby allowing a high amount of free calcium ions when MBP is capturing the target structure.

In addition to, or instead of an running buffer, an extraction buffer or a chase buffer can be used, which comprises 100 to 500 mM free calcium ions, or an amount of free calcium ions which complements an amount of free calcium ions that is already provided in the aqueous liquid (e.g., an aqueous extract) and/or within a device used for the LFA (such as an amount provided in a conjugate buffer), and optionally in a running buffer, to set a total amount of 100 to 500 mM free calcium ions in the CRB, thereby allowing a high amount of free calcium ions when MBP is capturing the target structure.

A typical buffer system comprises any one or more of an extraction medium, in particular an extraction liquid (e.g., an extraction buffer), a running buffer, optionally a chase buffer and/or an additional buffer at a conjugate pad, to obtain or set the desired composition of the CRB in the reaction zone of the LFA device, thereby supporting the capture reaction in a medium comprising or consisting of the CRB.

In particular, the CRB buffer is present, when MBP is binding to its target structure, first in a conjugate zone or pad where the MBP reporter nanoparticles are capturing the target structure, and second at the test region wherein immobilized MBP captures the target structure that is bound by the MBP reporter nanoparticles, thereby forming the MBP sandwiched capture product. There is optionally a third reaction at the control region, if a target structure of MBP (such as e.g., mannan) is used as a control (also called the control target structure), and where MBP reporter nanoparticles capture the control target structure.

Specifically, an MBP capture product comprises or consists of an MBP that binds to ("captures") the target structure, if present. As described herein, formation of an MBP capture product is specifically in the presence of the capture reaction buffer (CRB).

An MBP capture product is typically present in an area of a respective LFA device, where the MBP reporter nanoparticles (sometimes referred to as "conjugate") first come into contact with the target structure such as e.g., in a reaction zone which comprises a "conjugate zone" (also referred to as "conjugate release zone"). Such area may comprise the MBP reporter nanoparticles which bind to the target structure (if present) and move along an LFA membrane. Such MBP capture product is herein also referred to as a "first MBP capture product"

Typically, the first MBP capture product is allowed to flow across a membrane of an LFA device, where it aggregates along a test region such as a test line (which is also within the reaction zone, downstream of the conjugate zone), if the target structure or analyte is present.

An MBP capture product can be an MBP sandwiched capture product which comprises or consists of the target structure that is sandwiched by the MBP reporter nanoparticles, and the MBP of the test region or line. The MBP sandwiched capture product is typically present in an area of a respective LFA device, where a first MBP capture product comes into contact with another MBP such as e.g., at a test region, or on a test line. Said another MBP is typically an MBP that is adsorbed to or otherwise bound ("immobilized") to the test region or test line of the membrane.

A control line is typically used to verify if the test is working properly and/or to help quantify a test line.

An MBP capture product can also be present in an area of a respective LFA device, where MBP reporter nanoparticles come into contact with a control target structure such as e.g., mannan on a control line where the MBP reporter nanoparticles aggregate upon binding to the control target structure. Such MBP capture product is herein also referred to as a "control MBP capture product".

Alternatively, a microbe-structure independent ligand and ligand-specific target can be used as a control (i.e. a test control, which is not mannose-specific), wherein the ligand is provided as a nanoparticle-conjugate at the conjugate zone and the ligand-specific target is immobilized at the lateral flow membrane.

Specifically, a CRB is used during all MBP capture reactions, in particular during:
(i) the formation of a first MBP capture product; specifically, such first MBP capture product consists of the MBP reporter nanoparticles, which MBP is reacting with (in particular binding to, or bound by) the target structure, if present;
(ii) the formation of an MBP sandwiched capture product; specifically, such MBP sandwiched capture product consists of the first MBP capture product, which first MBP capture product is further bound to the test region or test line, which comprises immobilized MBP that is reacting with (in particular binding to, or bound by) the target structure of the first MBP capture product; and
(iii) optionally, the formation of the control MBP capture product; specifically, such control MBP capture product consists of the MBP reporter nanoparticles that are reacting with (in particular binding to, or bound by) a control target structure.

With this LFA and respective LFA setup or configuration, it is possible to detect 1 cfu per 100 ml of extracted oily liquid of most microorganisms in fuel tanks (i.e. a detection limit of 1 cfu/100 ml). It was found that the sensitivity was far improved with respect to a broad spectrum of microorganisms, to cover at least indicator microorganisms which are any one or more (or all) of Methylobacterium sp., Burkholderia sp., Rhodococcus erythropolis, Hormoconis resinae, Bacillus pumilus, Yarrowia lipolytica, or Candida tropicalis.

Specifically, a recombinant cell line is used to express MBP. Such recombinant MBP is preferably used in the LFA described herein.

Specifically, a preparation of MBP is used which comprises less than 10% of monomeric MBP. Specifically, a preparation of MBP is used which comprises less than 10% of MBP multimers (including e.g., dimers, trimers or oligomers) of 140 kDa or higher than 140 kDa. Preferably, the majority of MBP in the preparation is comprised as multimers smaller than 140 kDa, preferably MBP dimers or trimers. Specifically, the multimers smaller than 140 kDa are MBP dimers or trimers.

The multimerization of MBP has been associated with increased avidity for multivalent ligands, allowing for enhanced binding to mannose-rich material from microorganisms.

According to a specific aspect, MBP is comprised in an MBP preparation which comprises MBP multimers, wherein 10%-90% of the MBP is comprised as multimers smaller than 140 kDa, preferably as MBP dimers and/or trimers.

Specifically, the MBP is a mannose-binding lectin (MBL), such as a human MBL or non-human mammalian MBL. MBL is a preferred MBP as described herein.

Preferably, the MBP or MBL
a) comprises at least one mannose-binding C-type lectin domain (CTLD), preferably wherein said MBP comprises at least 90% sequence identity to any one of SEQ ID NO:1-6, or a CTLD of any of the foregoing, or
b) the MBP comprises or consists of a CTLD which comprises SEQ ID NO:15.

Specifically, a preparation of MBP or MBL is used which comprises less than 10% of monomeric MBL. Specifically, a preparation of MBL is used which comprises less than 10% of MBL multimers (including e.g., dimers, trimers or oligomers) of 140 kDa or higher than 140 kDa. Preferably, the majority of MBL in the preparation is comprised as multimers smaller than 140 kDa, preferably MBL dimers or trimers. The multimerization of MBL has been associated with increased avidity for multivalent ligands, allowing for enhanced binding to mannose-rich material from microorganisms.

According to a specific aspect, MBL is comprised in an MBP (or MBL) preparation which comprises MBL in the form of MBL multimers, wherein 10%-90% of the MBL is comprised as multimers smaller than 140 kDa, preferably as MBL dimers and/or trimers.

Specifically, the enhanced binding of the MBP (or MBL) to mannose-rich material from microorganisms provides for a particular improvement of the LFA described herein such as avoiding or reducing fading of a test line during a period of at least 30 minutes following its formation.

According to a specific aspect, the oily liquid is an oil or oily aqueous condensate or emulsion, preferably wherein the oily liquid originates from crude oil, petroleum, mineral oil, or a fraction of any of the foregoing, or a fuel such as kerosene, petrol, diesel, or biodiesel. Specifically, the oily liquid is a fuel.

Specifically, the oily liquid can originate from or be an oil or grease from plants (such as biodiesel), microorganisms or animals.

According to a specific aspect, the aqueous liquid is prepared by extracting the oily liquid using an extraction liquid, in particular an extraction buffer. The aqueous liquid may comprise the extraction liquid or buffer. Specifically, the aqueous liquid comprising the extraction liquid or buffer can be directly used in the LFA, such as by direct application of the aqueous liquid onto a sample zone (e.g., applied on a sample pad) of an LFA device.

According to a specific embodiment, the extraction buffer comprises 100 to 500 mM free calcium ions, a detergent, a buffer agent and a pH ranging between 6.5 and 9, preferably a pH of about (i.e., +/- 0.5 or +/- 0.25) 7 or 8.

Specifically, the CRB does not comprise a calcium-chelating agent such as EDTA.

Specifically, the buffer system does not comprise a calcium-chelating agent such as EDTA.

Specifically, one or more different buffer substances can be present in the CRB, such as Phosphate, Borate, MES, HEPES or Tris.

Specifically, one or more different detergents can be present in the CRB, such as NP-40, Triton-X100, Tween 20, Tween-80, CHAPS, CA-630, Brij-35, Triton-X114, GDN, GLC and Pluronic-F68.

Additional substances included in the buffer system or CRB may be sugars (such as sucrose, trehalose), PVP (polyvinylpyrrolidone) or PVA (polyvinylacetate).

According to a specific aspect, the CRB is used as running buffer of the LFA.

According to a specific aspect, mannan is used as a positive control.

Specifically, mannan is used at a control region of the lateral flow membrane, such as on a control line, in particular by immobilizing on the control line or region of an LFA device.

Specifically, a mannan is used which is a highly branched complex carbohydrate, an α-1,6 backbone with α-1,2- and α-1,3-attached mannose side chains, such as e.g., yeast mannan.

Alternatively, other mannose-containing compounds can be used as a positive control, and can, thus, be used at a control region or on a control line.

Alternatively, a mannose-independent, or microbe-structure independent ligand and a respective ligand-specific target are used as a control (e.g., a test control), wherein the ligand is provided as a nanoparticle-conjugate at the conjugate zone and the ligand-specific target is immobilized at the lateral flow membrane.

According to a specific aspect, a compound bound to reporter nanoparticles can be used in combination with the MBP reporter nanoparticles in the LFA, which is used for reacting with a control that specifically binds to such compound. Such compound can e.g., be an antibody or an antigen that is not interfering with the MBP reaction, and the respective positive control is another reactant (e.g., another antibody) that specifically binds to the respective antibody or antigen on a control line.

According to a specific aspect, the MBP is a protein comprising a lectin domain (such as a lectin domain of a C-type lectin) that recognizes in a Ca²⁺-dependent manner mannose-containing structures present on glycoproteins, such as ICAM-2 and ICAM-3.

Specifically, the MBP is selected from a mannose-binding lectin (MBL), Dendritic cell-specific ICAM-3 grabbing non-integrin (DC-SIGN e.g., of R&D Systems, USA, catalog number 9136-DC), Dectin-2 (e.g., R&D Systems, USA, catalog number 3114-DC), MMR (e.g., R&D Systems, USA, catalog number 2534-MR) or Langerin (e.g., R&D Systems, USA, catalog number 2088-LN).

Preferably, the MBP comprises or consists of a C-type lectin.

According to a specific aspect, the MBP comprises or consists of at least one naturally-occurring mannose binding C-type lectin or respective C-type lectin domain, or a recombinant protein comprising or consisting of the amino acid sequence of such lectin or lectin domain.

Preferably, the MBP is a mannose-binding lectin (MBL) such as comprising or consisting of a mannose binding C-type lectin or respective C-type lectin domain, preferably a human MBL or non-human mammalian MBL.

An exemplary lectin is human MBP, or an MBP from other eukaryotic origin, in particular MBP comprising or consisting of any one of SEQ ID NO:1-6, or the CTLD of any of the foregoing. Specifically, the MBP is human-derived or non-human animal derived, such as originating from mammalian species e.g., human, mouse, or bovine species, preferably human MBP or MBL.

Specific examples are
a) human MBP comprising SEQ ID NO:1 (Uniprot Database no. PRO_0000017401, 21-248, Mannose-binding protein C), SEQ ID NO:6, or SEQ ID NO:10;
b) murine MBP comprising SEQ ID NO:2 (Uniprot Database no. PRO_0000017413), SEQ ID NO:3 (Uniprot Database no. PRO_0000017405), SEQ ID NO:11, or SEQ ID NO:12;
c) bovine MBP comprising SEQ ID NO:4 (Uniprot Database no. PRO_0000017397), or SEQ ID NO:13;
d) human CD209 comprising SEQ ID NO:5 (Uniprot Database no. PRO_0000046595, human DC-SIGN), or SEQ ID NO:14; or
e) a functional variant of any of the foregoing which is capable of binding to the target structure, and optionally characterized by a certain sequence identity to any one of the respective sequences, or at least the respective CTLD thereof.

Specifically, the MBP comprises or consists of at least any one of 90%, 95%, 96%, 97%, 98%, 99%, or 100% sequence identity to any one of SEQ ID NO:1-6, or at least any one of 90%, 95%, 96%, 97%, 98%, 99%, or 100% sequence identity to a CTLD of any of the foregoing.

Specifically, the MBP comprises or consists of a C-type lectin domain of any one of SEQ ID NO:10-14, and at least any one of 90%, 95%, 96%, 97%, 98%, 99%, or 100% sequence identity to any one of SEQ ID NO:10-14, preferably wherein the MBP comprises at least any one of 90%, 95%, 96%, 97%, 98%, 99%, or 100% sequence identity to any one of SEQ ID NO:1-6.

According to a specific aspect, the MBP comprises any one of SEQ ID NO:10-14, and at least any one of 90%, 95%, 96%, 97%, 98%, 99%, or 100% sequence identity to any one of SEQ ID NO:1-6.

SEQ ID NO:15 is a consensus sequence of a mannose-binding CTLD. According to a specific aspect, the MBP comprises or consists of
a) a CTLD which comprises or consists of SEQ ID NO:15, or
b) a CTLD which comprises or consists of SEQ ID NO:15 and any one of at least any one of 90%, 95%, 96%, 97%, 98%, 99%, or 100% sequence identity to any one of SEQ ID NO:10-14; or
b) a CTLD which comprises or consists of SEQ ID NO:15 and any one of at least any one of 90%, 95%, 96%, 97%, 98%, 99%, or 100% sequence identity to any one of SEQ ID NO:1-6;

Preferably, the MBP comprises SEQ ID NO:15, and at least any one of 90%, 95%, 96%, 97%, 98%, 99%, or 100% sequence identity to any one of SEQ ID NO:1-6.

Specifically, the MBP is a recombinant protein expressed in eukaryotic or procaryotic expression systems.

Exemplary expression systems are mammalian host cells preferably a human animal-derived or non-human animal derived host cell, preferably a host cell selected from the group consisting of Chinese hamster ovary (CHO)-cell lines, mouse myeloma (NS0)-cell lines, HEK-293, HT1080, H9, HepG2, MCF7, MDBK Jurkat, MDCK, NIH3T3, PC12, BHK (baby hamster kidney cell), VERO, SP2/0, YB2/0, Y0, C127, L cell, COS, e.g., COS1 and COS7, QC1-3, VERO, PER.C6, HeLA, EBI, EB2, EB3, or hybridoma-cell lines.

Alternative expressions systems are insect, plant, yeast or bacteria.

Specifically, the MBP is a recombinant protein expressed in E. coli or other non-glycosylating expression systems.

According to a specific aspect, said MBP is comprised in an MBP preparation which comprises MBP multimers, wherein 10%-90% (w/w) of the MBP, preferably at least any one of 10%, 20%, 30%, 40%, 50%, 60%, 70%, or 80% (w/w) of the MBP, is comprised as multimers including e.g., dimers, trimers and/or oligomers.

Specifically, the MBP multimers are smaller than 140 kDa, preferably comprised in the MBP preparation as dimers and/or trimers.

Specifically, the majority of the MBP molecules (e.g., at least any one of 50%, 60%, 70%, or 80% (w/w)) comprised in the MBP preparation are MBP dimers or trimers.

Specifically, the amount of MBP monomers and MBP multimers, in particular dimers and trimers can be determined by non-reducing SDS PAGE.

According to a specific example, a human MBP preparation is used, wherein the MBP dimers or trimers have a size of approximately 55 and 85 kDa, respectively.

According to a specific aspect, the same MBP preparation is used on both, at the test region and on the MBP reporter nanoparticles. Specifically, the MBP preparation immobilized at the test region, and bound to the reporter nanoparticles is of the same quality or source.

According to a specific aspect, the reporter nanoparticles comprise or consist of a tracer. Specifically, the tracer has a distinctive optical property. Specifically, the reporter nanoparticles comprise a tracer, such as a visually identifiable tracer, which allows visual identification of an MBP capture product. Specifically, the MBP sandwiched capture product is visually identifiable by using such tracer. If a control region or line is used, the MBP capture product at the control region or line can be visually identifiably as well.

Specifically, detecting the presence of tracers, in particular coloured or fluorescent MBP containing reporter nanoparticles at the test line and optionally at the control line of an LFA device, can be done visually under natural lighting. Visual reading of lateral flow tests may not be possible for fluorescent tracers or other tracers that are not visually identifiable, for which a respective reader can be conveniently used.

Specifically, the tracer comprises or consists of gold, silver, platinum, carbon, fluorescence, coloured latex or magnetic tracer, preferably colloidal gold.

Specifically, the reporter nanoparticle is dyed latex. In an embodiment, the dyed latex nanoparticle is between about 100 nm and about 200 nm in size. In an embodiment, the dyed latex nanoparticle is about 150 nm in size.

Specifically, the reporter nanoparticle is colloidal gold such as a gold nanoparticle or a gold nanoshell. In an embodiment, the gold nanoparticle or the gold nanoshell is between about 10 nm and about 80 nm in size. In an embodiment, the gold nanoparticle or the gold nanoshell is about 40 nm in size.

According to a specific aspect, the microbial contaminants are any one or more microorganisms, such as bacteria, yeast, fungi, or spores. Specifically, the microorganisms comprise a glycan structure that can be recognized and bound by MBP, in particular a target structure, preferably mannan or a mannose containing structure. Some MBPs can also additionally recognize other glycan structures such as N-acetylglucosamine, fucose, glucose, as well as lipopolysaccharides (LPS).

Specifically, the method described herein is used to determine microbial contaminants from mannose-containing microorganisms, in particular those which can grow in oily liquids such as fuel (e.g., diesel, kerosene, or biodiesel), and thereby contaminate such oily liquids.

Contaminants may originate from target microorganisms such as diesel, kerosene, or biodiesel contaminating microorganisms. Exemplary microorganisms are selected from Trichocomaceae, Saccharomycetaceae, Acetobacteraceae, Thermotogae, Spirochaetes, Firmicutes, Bacteroidetes, Clostridiaceae, Lentisphaerae, Thermoprotei, Methanocellales, Methanosarcinales, Methanosaeta, Methanoculleus, alpha-proteobacteria, beta-proteobacteria and gamma proteobacteria, in particular the genera Methylobacteria, Pseudomonas, Kocuria, Amorpotheka, Aspergillus, Fusarium, Penicillium, Methylobacterium, Bacillus, Paenibacillus, Micrococcus, Brachybacterium, Aerococcus, Zimmermannella, Serratia, Sphingomonas, Variovorax, Flavobacterium, Arthrobacter, Amorphotheca and Alcaligenes

Specifically, target microorganisms comprise any one or more of the following: Methylobacterium sp., Burkholderia sp., Rhodococcus erythropolis, Hormoconis resinae, Bacillus pumilus, Yarrowia lipolytica, or Candida tropicalis.

Specifically, indicator microorganisms are used to determine the sensitivity and robustness of the LFA, such as e.g., any one or more of the following: Methylobacterium sp., Burkholderia sp., Rhodococcus erythropolis, Hormoconis resinae, Bacillus pumilus, Yarrowia lipolytica, or Candida tropicalis.

According to a specific aspect, the method detects the microbial contaminants with a sensitivity of 1 cfu per 100 ml of the oily liquid, or higher.

Specifically, the detection limit can be about (+/-1) 10 cfu/100ml, preferably about 9, 8, 7, 6, 5, 4, 3, or 2, or can be 1 cfu per 100 ml.

According to specific examples, the LFA is carried out as follows.
a) The oily fluid is treated to prepare an aqueous liquid, such as e.g., by preparing an aqueous extract, or by liquid-liquid separation to separate an aqueous condensate. To prepare the aqueous liquid, an extraction liquid can be used which may comprises all or part of the amount of free calcium ions, as required for the CRB described herein.
b) The aqueous liquid is applied onto an LFA device e.g., within a sample zone of the device, in particular to a sample pad.
c) The sample flows through a reaction zone of the LFA device where glycan-containing microbial contaminants, if present, can first be captured by identifiable (e.g., by using an identifiable tracer) MBP-containing reporter nanoparticles, such as MBP adsorbed to colloidal gold, thereby forming a first MBP capture product. For this first capture reaction, a CRB described herein is used as a reaction buffer.
d) The first MBP capture product is moving to a detection zone within the reaction zone of the LFA device where MBP is immobilized (e.g., adsorbed) at a test region, such as on a test line, wherein the immobilized MBP captures the first MBP capture product, in particular through binding to the microbial contaminants that are present in the first capture MBP product, thereby forming an MBP sandwiched capture product. Such MBP sandwiched capture product that is bound to the test region or line via the immobilized MBP, can be identified either visually or by an identifying reader, depending on the tracer used for identification. For this second capture reaction, again the CRB is used as a reaction buffer.

The LFA device is conveniently configured to allow a capillary flow through the LFA device using the buffer system described herein.

Optionally, the flow of the MBP-containing reporter nanoparticles through the LFA device continues to get to a control region or line, which comprises an immobilized (e.g., through adsorption) positive control, such as a target structure e.g., mannan, thereby forming another MBP capture product, in particular through binding the MBP reporter particles to the immobilized target structure, which MBP capture product can be identified either visually or by an identifying reader, depending on the tracer used for identification.

Optionally, the flow of the buffer through the LFA device ends at a wick or wicking pad (absorption pad), to absorb the liquid remainders.

The invention further provides for an analytical test kit for detecting microbial contamination in an oily liquid, in particular a sample of an oily liquid, comprising:
a) a lectin-based lateral-flow assay (LFA) device, which employs mannose-binding protein (MBP) as capture agent that is bound to flowable, identifiable reporter nanoparticles at a reaction zone of a lateral flow membrane; and
b) a running buffer which comprises a detergent, a buffer agent and a pH ranging between 6.5 and 9.0, and which sets an amount of 100 to 500 mM free calcium ions at the reaction zone.

Specifically, the invention provides for an analytical test kit for detecting microbial contamination in an oily liquid, comprising:
a) a lectin-based lateral-flow assay (LFA) device, which employs mannose-binding protein (MBP) as capture agent that is bound to flowable, identifiable reporter nanoparticles at a reaction zone of a lateral flow membrane; and
b) a running buffer which comprises a detergent, a buffer agent and a pH ranging between 6.5 and 9.0, which running buffer comprises 100 to 500 mM free calcium ions and sets an amount of 100 to 500 mM free calcium ions at the reaction zone.

Specifically, the reaction zone comprises a first reaction zone (also referred to as "conjugate zone") where the MBP reporter nanoparticles are placed e.g., in the presence of a conjugate buffer.

Specifically, the reaction zone comprises at least the first reaction zone and a second reaction zone (also referred to as "detection zone") where MBP is immobilized at or within a test region or on a test line, for use as a capture agent at the test region or line.

Specifically, the reaction zone comprises compounds and buffers in the dry form. According to a specific aspect, the running buffer comprises:
a) 100 to 500 mM free calcium ions; or
b) an amount of free calcium ions which complements an amount of free calcium ions that is provided within the LFA device e.g., provided in any one or more of the zones or regions of the LFA device; or
c) an amount of free calcium ions which complements an amount of free calcium ions that is provided within the sample (or the aqueous liquid that is prepared from the oily liquid), which is applied to the LFA device, and/or an amount of free calcium ions that is provided within the sample that is applied to the LFA device and/or an amount of free calcium ions that is provided within the LFA device e.g., provided in any one or more of the zones of the LFA device, such as the reaction zone.

In addition to the running buffer, the kit may further comprise an extraction buffer. Specifically, an extraction liquid (e.g., an extraction buffer) can be used to prepare an aqueous extract from the oily liquid.

Specifically, the running buffer can also be used as an extraction buffer for extracting the oily liquid.

Specifically, the running buffer is an extraction buffer.

Specifically, the kit may comprise an extraction buffer that is also used as a running buffer. The extraction buffer is preferably characterized by the same features as described herein for a running buffer.

According to a specific embodiment, the test kit comprises an extraction buffer instead of the running buffer.

Specifically, a buffer system of an LFA is provided with the test kit, which comprises or consists of any one or more of a running buffer, an extraction liquid (e.g., an extraction buffer), a reaction buffer (CRB), a conjugate buffer, or chasing buffer.

Specifically, a buffer system is provided with the test kit, to set an amount of free calcium ions in the capture reaction buffer (CRB), in particular in the CRB that is present in the reaction zone of the LFA device.

Specifically, a buffer system is provided with the test kit to set an amount of 100 to 500 mM free calcium ions, a detergent, a buffer agent and a pH ranging between 6.5 and 9.0 in the CRB, in particular in the CRB that is present in the reaction zone of the LFA device.

Specifically,
a) the running buffer comprises one or more different detergents selected from the group consisting of NP-40, Triton-X100, Tween 20, Tween-80, CHAPS, CA-630, Brij-35, Triton-X114, GDN, GLC and Pluronic-F68; and
b) the running buffer comprises one or more different buffer substances selected from the group consisting of Phosphate, Borate, MES, HEPES or Tris.

Specifically, the running buffer (or extraction buffer, if used as a running buffer) comprises 100 to 500 mM free calcium ions, or an amount of free calcium ions which complements an amount of free calcium ions that is provided in the buffer system of the LFA and/or within the LFA device, to set a total amount of 100 to 500 mM free calcium ions when MBP is capturing the target structure.

Specifically, a running buffer is used which comprises 100 to 500 mM free calcium ions, or an amount of free calcium ions which complements an amount of free calcium ions that is provided in an extraction buffer and/or within the LFA device, to set a total amount of 100 to 500 mM free calcium ions when MBP is capturing the target structure.

According to a specific aspect, the reaction zone is configured to receive a sample, e.g., as the flow passes through a sample zone and moves forward to the reaction zone. Specifically, the reaction zone comprises the MBP bound to the reporter nanoparticles at a conjugate zone, and a detection zone, such as a detection zone that is downstream the conjugate zone, which detection zone comprises a test region, preferably wherein the test region comprises a test line.

Specifically, the reaction zone comprises the MBP bound to the reporter nanoparticles at a conjugate zone, and a test region comprising a test line where immobilized MBP is used as a capture agent, and optionally a control area or line where a control is immobilized, such as a positive control or test control.

According to a specific aspect, the test kit is characterized by one or more features as described herein with respect to the LFA or analytical method.

According to a specific aspect, the kit is characterized by one or more features as described herein with respect to the analytical method and the LFA components, such as e.g., the features of the MBP.

Preferably, the MBP is comprised in an MBP preparation which comprises MBP multimers, wherein 10%-90% of the MBP is comprised as multimers smaller than 140 kDa, preferably wherein the multimers smaller than 140 kDa are dimers and/or trimers.

Preferably, the MBP is a mannose-binding lectin (MBL).

Specifically, said MBP or MBL
a) comprises at least one mannose-binding C-type lectin domain (CTLD), preferably wherein said MBP comprises at least 90% sequence identity to any one of SEQ ID NO:1-6, or a CTLD of any of the foregoing, or
b) the MBP comprises or consists of a CTLD which comprises SEQ ID NO:15.

Specifically, the LFA device comprises one or more of the following features:
a) the reporter nanoparticles comprise or consist of a tracer, preferably a gold, silver, platinum, carbon, fluorescence, colored latex or magnetic tracer, preferably colloidal gold.
b) the lateral flow membrane is cellulose-based or synthetic fiber-based, preferably a nitrocellulose membrane or other cellulose-based paper;
c) the same MBP preparation is used as capture agent that is bound to the reporter nanoparticles and on the test line;
d) the MBP at the test region or on the test line is immobilized in or on the lateral flow membrane and resides at a fixed part of the membrane;
e) mannan is used as positive control, or a microbe-structure independent ligand and ligand-specific target are used as a test control, wherein the ligand is provided as a nanoparticle-conjugate at the conjugate zone and the ligand-specific target is immobilized at the lateral flow membrane.

Specifically, a binding agent described herein (e.g., the MBP or a control) is immobilized in or on the lateral flow membrane through physical adsorption, chemical binding, or affinity binding e.g., by:
i) dispersion of the agent within a certain area of the membrane and direct physical adsorption on the membrane, such as by drying onto the membrane; or
ii) covalent attachment of the agent to an epoxide-functionalized membrane, such as a nitrocellulose membrane; or
iii) attachment of a biotinylated agent through a membrane-binding streptavidin anchor protein, such as a protein anchored to nitrocellulose membrane.

The invention further provides for the use of a test kit described herein in the analytical method described herein.

The invention further provides for the method described herein, wherein a test kit of described herein is used.

Specifically, the test kit is used with a buffer system which sets an amount of 100 to 500 mM free calcium ions, a detergent, a buffer agent and a pH ranging between 6.5 and 9.0 in the CRB. Specifically, the CRB is used during one or more of the reactions of the capture agent (i.e. the MBP) to bind the target structure.

The invention further provides for a method of reducing or avoiding fading of the intensity of a test signal in a reaction zone of a lateral flow membrane of a lectin-based lateral-flow assay (LFA) device, which assay determines the presence of glycans in an aqueous liquid produced from an oily sample liquid by reacting with a mannose-binding protein (MBP) as capture agent in the reaction zone using a capture reaction buffer (CRB) which comprises 100 to 500 mM free calcium ions, a detergent, a buffer agent and a pH ranging between 6.5 and 9.0.

Specifically, fading or the degree of fading of the test signal is reduced to less than any one of 50%, 40%, 30%, 30%, 20%, or 10% loss of intensity of the signal within at least any one of 30, 40, 50, or 60 minutes following formation of the test signal.

Fading is considered to be avoided when fading is reduced to less than 10% signal intensity, or when no fading can be visibly determined by viewing i.e., no fading is visible to the eye of the person carrying out the LFA.

Specifically, fading is reduced or avoided within at least 30 minutes following producing the test signal.

Fading or the degree of fading can be determined at room temperature, in particular in the environment of a room or laboratory where the LFA is carried out.

Specifically, the method of reducing or avoiding fading employs an assay that detects microbial contaminants in an oily liquid according to the method described herein, or wherein a test kit described herein is used.

All features as disclosed with respect to the analytical method described herein also apply to the method of reducing or avoiding fading as described herein, as appropriate, and *vice versa.*

All features as disclosed with respect to the methods (in particular any one or both of the analytical method described herein and method of reducing or avoiding fading as described herein) also apply to the test kit described herein, as appropriate, and *vice versa.*

### FIGURES

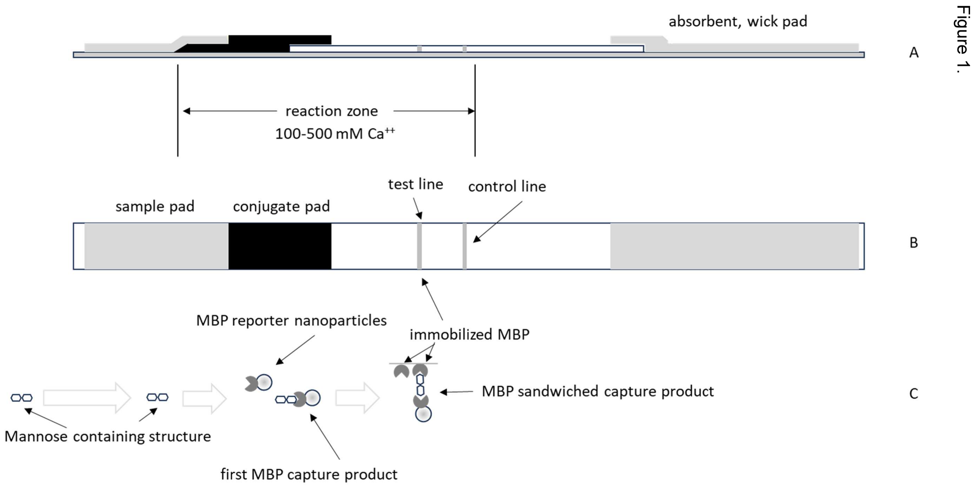

Figure 1. Schematic representation of a typical lateral flow assay described herein. A) shows a side view of the LFA strip according to the invention, B) shows a top view of the strip, C) shows the flow of a target molecule, a mannose containing structure, which is captured in the reaction zone to form a first capture product under conditions as described in the invention and a sandwiched product which is responsible for the signal at the test line.
Figure 2: Sequences provided herein.

### DETAILED DESCRIPTION

Unless indicated or defined otherwise, all terms used herein have their usual meaning in the art, which will be clear to the skilled person. Reference is for example made to the standard handbooks, such as Sambrook et al., 2012, Molecular Cloning: A Laboratory Manual, volumes 1-4, Cold Spring Harbor Press, NY); Lewin, "Genes IV", Oxford University Press, New York, (1990), and Janeway et al., "Immunobiology" (5th Ed., or more recent editions), Garland Science, New York, 2001, Ausubel et al., Current Protocols in Molecular Biology, John Wiley and Sons, Baltimore, Md. (1989), Vega et al., Gene Targeting, CRC Press, Ann Arbor Mich. (1995); Vectors: A Survey of Molecular Cloning Vectors and Their Uses, Butterworths, Boston Mass. (1988); "The Immunoassay Handbook": Wild, D. (2013), ISBN: 978-0-08-097037-0; and "Lateral Flow Immunoassay" by Raphael Wong and Harley Tse (2009), Humana Press, ISBN: 978-1-58829-908-6.

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

Unless further specified herein, the term "about" or "around" as used herein refers to the same value or a value differing by +/-10% or +/-5% of the given value.

Specific terms as used throughout the specification have the following meaning.

The term "aqueous liquid" as used herein refers to a liquid which is water-based, such as an aqueous solution or suspension of compounds in water. For an LFA described herein, typically, an aqueous liquid comprising the target analyte is prepared from an oily liquid, and applied to an LFA for determining the analyte.

The term "free calcium ions" or "Ca⁺⁺" as used herein refers to calcium ions that are not complexed with any molecule or compound, e.g., a chelating agent, which can hinder its reaction with other molecules or ions to mediate binding of carbohydrate patterns on a microbial cell surface to a microbe surface-binding domain (e.g., MBL) of the engineered microbe-binding molecule. Specifically, free calcium ions can be present in the absence of chelating agent. In some embodiments, free calcium ions can be present in a solution comprising a chelating agent and calcium ions, wherein the amount of calcium ions present in the solution is at least about 30%, 40% or 50% more than an amount sufficient to interact with substantially all the chelating agent molecules present in the solution to form chelate complexes.

Specifically, free calcium ions can be obtained from a water-soluble calcium salt, which is particularly understood as a calcium salt which has significant solubility in water at room temperature, for example at least 1 gram per 100 ml water, at least 10 grams per 100 ml water, or at least 25 grams per 100 ml water or higher. Examples of calcium salts include, without limitations, calcium chloride, calcium fluoride, calcium bromide, calcium iodide, calcium nitrate, calcium citrate, calcium formate, calcium acetate, calcium gluconate, calcium ascorbate, calcium lactate, calcium glycinate and mixtures thereof. Specifically, calcium chloride can be used as a source of calcium ions.

The term "oily liquid" as used herein refers to a liquid which is an oil, oil-based liquid, or the oily phase of an emulsion. Also, an aqueous condensate from an oil or an emulsion of an oily phase with an aqueous phase can comprise hydrophobic substances from an oil, and are, thus, also considered as an oily liquid. Oily liquids can consist of or originate from crude oil, petroleum, mineral oil, or a fraction of any of the foregoing, preferably a fuel such as kerosene, petrol, diesel, or can originate from plants (e.g., biodiesel) or from microorganisms or algae.

The term "lateral-flow assay" abbreviated as LFA, is herein understood as a method of determining an analyte or respective test, using a lateral flow device, also referred to as an LFA device. A lectin-based LFA is understood as an LFA that employs a lectin as capture agent.

The term "LFA device" is herein understood to refer to a strip, cartridge, or cassette comprising one or more LFA test strips for detecting a target structure (i.e. an analyte) in a test sample, such as a glycan structure (e.g., mannose or mannan) that is recognized by a lectin like an MBP.

A typical LFA device or test strip described herein comprises a lateral flow membrane. Specifically, it comprises a sample zone for receiving the aqueous liquid; a reaction zone comprising the sample zone, overlapping with or downstream from the sample zone, the reaction zone comprising MBP containing reporter nanoparticles at a conjugate zone, which specifically bind to the target analyte, and a detection zone downstream of the conjugate zone; the detection zone comprises a test region or line comprising an immobilized MBP as capture agent that specifically binds to the target analyte, and optionally a control region or line comprising an immobilized control compound that is bound by MBP containing reporter nanoparticles, (or bound by other reporter nanoparticles if used in a mixture with the MBP reporter nanoparticles), wherein the control line region is within the detection zone, typically downstream of the test region or line,; and an absorbent zone downstream of the detection zone for capturing excess fluid, also referred to as "wicking pad", "wick pad", or "absorbance pad". By "downstream", it is meant to refer to a location later in the fluid flow path than one or more other locations.

The term "sample zone" or "sample receiving zone" as used herein, refers to the region of the LFA device where a test sample, such as the aqueous liquid as used herein, is deposited for fluid flow along an LFA strip. The sample receiving zone may be of any size or shape. The sample receiving zone is located upstream in a fluid flow path from the detection zone and the absorbent zone. The sample receiving zone may overlap with or be located upstream of the reaction zone. Specifically, the sample receiving zone is located upstream of the reaction zone. By "upstream", it is meant to refer to a location earlier in the fluid flow path than one or more other locations. The sample receiving zone may be made of any suitable material to receive the test sample and permit fluid flow along the lateral flow membrane or test strip, e.g. by capillary action. In other words, "upstream" refers to a direction opposite the direction of liquid flow, while "downstream" refers to a direction that is in the direction of flow.

The sample zone may comprise or consist of a sample pad, being the first component of an LFA test strip that comes into contact with the sample. The sample pad can act as a filter or may comprise a filter, which prevents the passage of unwanted material from the sample or retains unwanted particulates while allowing the sample fluid containing the analyte to flow through the test strip.

The term "reaction zone" as used herein refers to a region of the LFA device in which the test sample, such as the aqueous liquid as used herein, is able to come into contact with the MBP containing reporter nanoparticles, thereby forming an MBP capture product. The reaction zone may be in the same region of the lateral flow membrane or test strip as the sample zone, or may partially overlap with the sample zone, or may be entirely downstream along a fluid flow path from the sample zone. The reaction zone typically comprises a conjugate zone which may comprise or consist of a conjugate release pad. Such conjugate release pad in an LFA typically holds and preserves the capture agents, in particular MBP bound to or conjugated to reporter nanoparticles which is used as a capture agent in the conjugate zone. As the sample flows through the conjugate zone, MBP reporter nanoparticles can be released into the sample fluid and can bind with the target analyte, if present. The sample and the MBP reporter nanoparticles can then flow together to the detection zone of the reaction zone.

The "detection zone" as used herein, refers to a region of the LFA device in which, by fluid flow, the first MBP capture product travel to and interact with the MBP immobilized on a test region or line (i.e. a test area), thereby forming the MBP sandwiched capture product. Optionally, the detection zone further comprises a control region or line. Within the detection zone, a control region or line is typically located downstream of the test region or line, to control proper functioning of the LFA by confirming fluid flow from the reaction zone to the region of the test strip where the control line is located.

As used herein, the term "control" refers to any compound, molecule, or substance that is immobilized onto the control line or region and which is recognized and bound by a ligand that is comprised in the medium flowing through the LFA device to verify that the test is working properly.

According to a specific example, the control is a target structure, in particular mannan, and the MBP of the MBP containing reporter nanoparticles is a ligand that recognizes and binds to the target structure.

Alternatively, a compound bound to reporter nanoparticles can be used in combination with the MBP reporter nanoparticles in the LFA, which is used for reacting with a positive control that specifically binds to such compound.

Alternatively, the control can be completely independent of the MBL/microbial target system. For example, a control nanoparticle ligand conjugate that is mixed with the MBL nanoparticle conjugate is released at the same time as the MBL nanoparticle conjugate and binds to the respective ligand-binding partner, which can be immobilized onto the control region after the liquid front has passed the test line.

According to a specific example, a microbe-structure independent ligand and ligand-specific target can be used as a control (i.e. a test control, which is not mannose-specific), wherein the ligand is provided as a nanoparticle-conjugate at the conjugate zone and the ligand-specific target is immobilized at the lateral flow membrane.

Specifically, the LFA device, test strip, or lateral flow membrane comprises only a single test line or region in the detection zone, and optionally a control line or region located downstream thereof. If the device, test strip, or lateral flow membrane described herein comprises additional identifiable reporter nanoparticles with lectins or binders other than MBP containing reporter nanoparticles specific for different target analytes, the device or test strip may also comprise two or more of the test lines, each with one of MBP or one of said other lectins or binders that are immobilized on the test lines. Test lines or regions can be used which comprise not only MBP, but also MBP in combination with other lectins or binders, which allows determining a combination of target analytes recognized by any one or more of the MBP or the other lectins or binders.

Specifically, a detection zone of an LFA device comprises one, two or more of the test lines or regions and the immobilized detector capture reagent of each of the test lines or regions specifically binds to one of the different analytes. In an embodiment, the detection zone comprises one, two or more of the test line or regions and the immobilized detector capture reagent of each of the test line or regions is the same.

An "extraction liquid" as used herein refers to an aqueous liquid, such as an aqueous solution (e.g., a buffer) or water, which is used to extract microbial material from oily fluids into an aqueous solution, thereby producing an aqueous extract. It may also comprise components (e.g., lytic enzymes, detergents) that lyse whole cells. The aqueous extract can be separated from the hydrophobic liquid after thorough mixing with the extraction liquid by centrifugation or gravity. The aqueous extract may be diluted with a concentrated sample buffer in order to get all necessary substances into the sample before applying a part of the sample to the sample zone of an LFA device.

A running buffer may be added to keep the lateral flow going. The flow speed can be improved or stabilized using a "chase buffer" which increases the liquid flow along the lateral flow membrane.

One option is to include buffer constituents (e.g., running buffer or CRB constituents) within the LFA membrane e.g., by using buffer constituents dried on a sample pad and/or by using a conjugate buffer comprising buffer constituents at a conjugate zone. This can eliminate or reduce the application of buffer constituents to the lateral flow membrane with the analyte containing medium (e.g., a sample buffer, or an extract buffer). The conjugate buffer is herein understood as the buffer in which the MBP reporter nanoparticle (herein also referred to as "conjugate") is applied to a conjugate zone, such as a conjugate pad.

The terms "running buffer" and "sample buffer" are not equivalent, although they both play important roles in lateral flow assays. A running buffer is used to buffer sample pH, minimize non-specific binding, neutralize interferents, and control flow speed in the assay. On the other hand, a sample buffer is used to prepare the sample for the assay, helping to solubilize the analyte, maintain its stability, and facilitate the flow of the sample through the test strip or along the lateral flow membrane. While both buffers contribute to the overall performance of the assay, they serve distinct purposes and are not interchangeable.

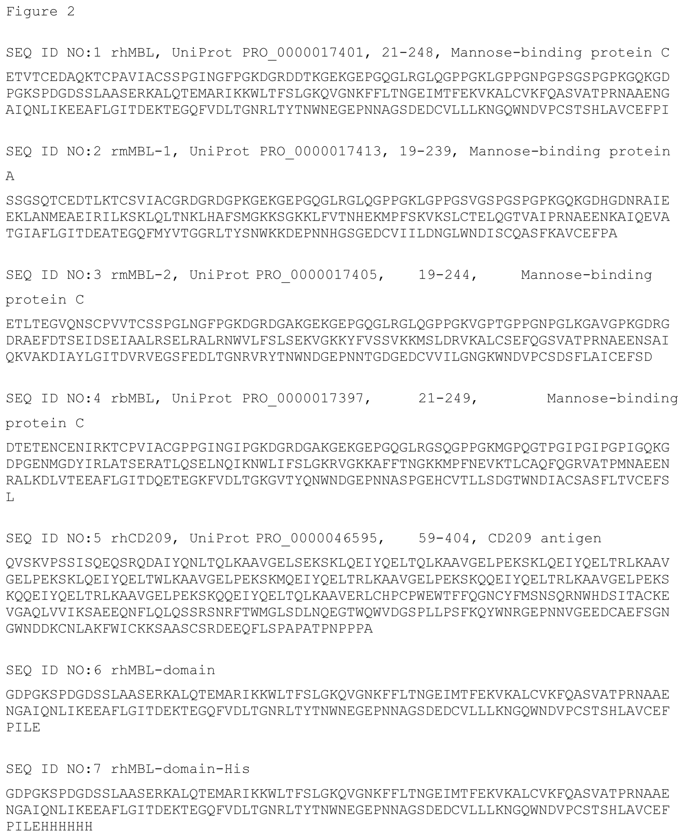

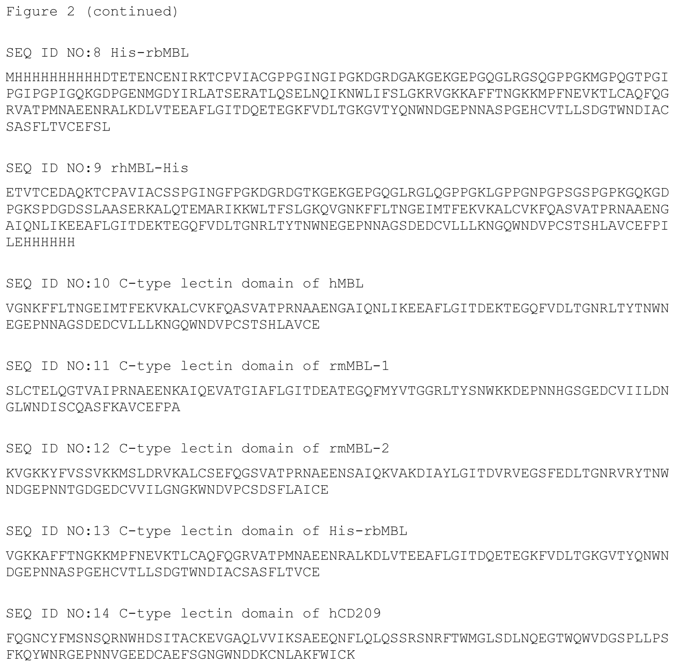

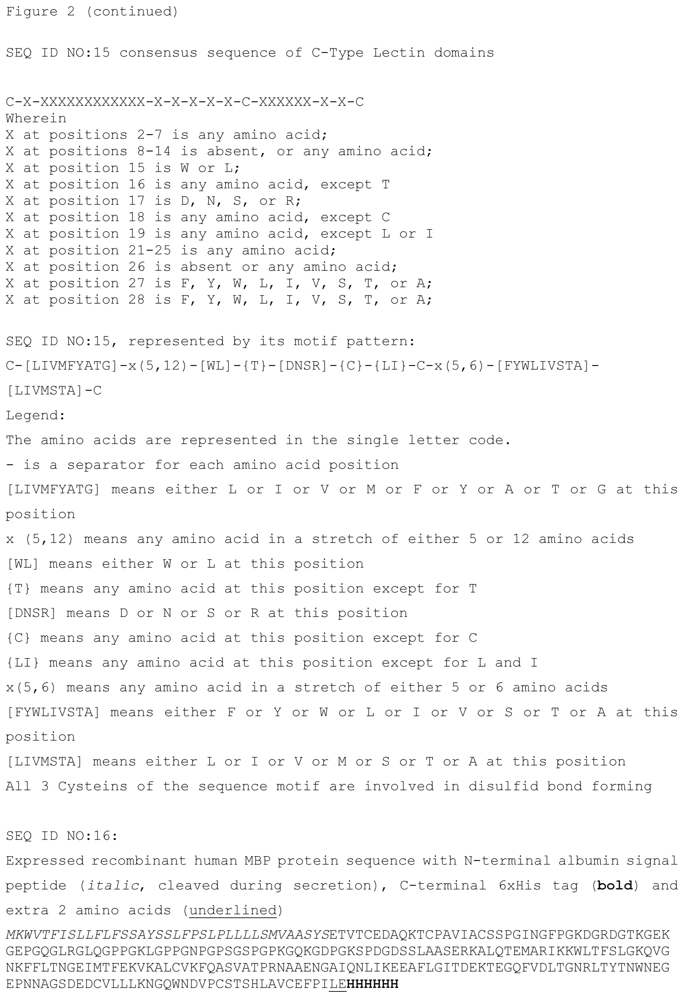

The term "mannose binding protein" (MBP) as described herein refers to a singular or a multimerized polypeptide, either natural or recombinant, which comprises at least one C-type-lectin domain capable of binding to mannose containing structures. The C-type lectin-like fold superfamily of proteins is classified as SCOP ID: 3001261 (Structural Classification of Proteins; Andreeva A, Kulesha E, Gough J, Murzin AG. The SCOP database in 2020: expanded classification of representative family and superfamily domains of known protein structures. Nucleic Acids Res. 2020;48(D1):D376-D382. doi:10.1093/nar/gkz1064). The C-type lectin domain signature and profile is described in PROSITE entry PS50041 (PROSITE database of protein families and domains, SIB Swiss Institute of Bioinformatics, Hulo N, Bairoch A, Bulliard V, et al. The PROSITE database. Nucleic Acids Res. 2006;34(Database issue):D227-D230. doi:10.1093/nar/gkj063). A consensus sequence of suitable C-type lectin domains is herein provided as SEQ ID NO:15. The respective consensus pattern is shown in Figure 2.

Depending on the molecular complexity of the recognized carbohydrates, two types of closely interlinked carbohydrate-binding specificities can occur at the carbohydrate-binding site of the MBP of the invention:
1. A mannose monosaccharide-binding specificity, allowing the MBP to specifically recognize mannose (Man) and its derivatives, e.g., methylmannoside. This type of mannose monosaccharide recognition by MBP corresponds to the "broad sugar-binding specificity" of lectins, which relies on the occurrence of a monosaccharide-binding pocket within the carbohydrate-binding site.
2. An oligosaccharide-binding specificity, which consists of the simultaneous accommodation of several sugar units of a complex N-glycan, e.g., high-mannose glycans, also known as the "fine sugar-binding specificity" of lectins. This type of oligosaccharide recognition involves most of the surface of the carbohydrate-binding site, including the monosaccharide-binding site.

Specifically, the MBP is functional in binding to the target structure, in particular specifically binding to the target structure.

Specifically, an MBP as used herein is characterized by its function to bind a mannose structure such as comprised in mannan, which can be determined in a standard ELISA using e.g., mannan comprising an α(1-6)-linked backbone and α(1-2)-linked and α(1-3)-linked branches, such as yeast mannan.

The term "mannan" as described herein refers to biopolymers of mannose, which are either linear polymers of mannopyranose residues connected in β-(1,4) bonds, or fungal cell wall mannan, which has an α(1,6) linked backbone decorated with α(1,2) and α(1,3) linked side branches. The term "mannan" includes glycomannans or mannan derivatives in reduced or oxidized forms. Generally, mannan is the generic name for the polysaccharide moiety of glycoproteins or mannoproteins. Mannan typically comprises a mannose structure that is targeted by MBP. Different types of mannans include galactomannans, glucomannans, galactoglucomannans, arabinomannan, mannan oligosaccharides, linear mannan, or branched mannan. Galactomannans typically contain mannose and galactose with β(1-4) linkages, occasionally with α(1-6) galactose branches. Glucomannans are found in yeast and have a different structure with a α(1-6) linked backbone and α(1-2) and α(1-3) linked glucose branches. Galactoglucomannans have a mixed mannose/glucose β(1-4) backbone. Mannan oligosaccharides (MOS) are the resulting smaller molecules when a long chain of mannan is hydrolyzed into shorter chains. They can be produced from either insoluble galactomannan or soluble glucomannan. Linear mannan is a linear polymer of mannopyranose residues.

Specific mannan compositions are yeast derived mannan. The cell wall of yeast is formed mostly of β-1,3-glucan, 1,6-glucan, mannan, and chitin. Yeast mannan contains an α(1-6)-linked backbone and α(1-2)-linked and α(1-3)-linked branches. Mannan found in the cell walls of Saccharomyces cerevisiae is composed of a linear chain of α-(1,6)-D-mannan decorated by side chains and attached to L-asparagine residues of proteins by a linkage unit consisting of two β-(1,4)-N-acetylglucosamine residues. The composition of the side chains varies, but largely consists of α-(1,3)-mannosyl-α-(1,2)-mannosyl-α-(1,2)-mannosyl chains. The side chain can be longer or shorter, and can be branched as well. In fungi, pure mannans are rarely found, and most contain α(1,6)-linked galactopyranosyl side chains. In certain filamentous fungi galactofuranose is found in galactomannan instead of galactopyranose. The galactomannan attached to N-glycans in Aspergillus fumigatus is composed of a linear mannan core comprising α-(1-2)-linked mannotetraose repeating units attached to each other via α-(1-6) linkages and decorated by side chains of β-(1-5)-galactofuranose units, which are linked to the C2 position of the non-reducing mannose residue in each mannotetraose unit.

Glycans are also constituents of the bacterial capsule, which may also include arabinomannan and mannan.

Mannan or Mannose can be used as a positive control in an MBP-based LFA. Specific control mannan preparations are mannan from Saccharomyces or high mannose glycoproteins.

The term "target structure" or "target analyte" as used herein, refers to any compound, molecule, or substance which is desired to be detected in a test sample. Target structures described herein are glycan structures of microbes such as bacteria, yeast, fungi or spores, which can be recognized and bound by MBP.

Specifically, the target structure as used herein is a mannose-containing structure, which is herein understood as any chemical structure comprising at least one mannose residue. Specific glycan structures recognized by MBPs are target mannose structures, such as comprised in mannose, oligosaccharides containing mannose, or mannan. Mannose-containing structures are e.g., included in mannans, glycoproteins or glycolipids, and can be comprised in glycan-containing microbial material, such as whole cells, spores, cellular debris, released products or secreted products.

The term "fading" as used herein, refers to a decrease of line intensity over time, such as a decrease of line intensity while performing an LFA and reading the control and optionally the test line (herein also referred to as signal line). Fading can particularly occur after initial formation of a signal line in a lateral flow assay. Fading may be observed in any of the lines of a lateral flow assay, i.e. signal line or control line. Depending on the time scale and the magnitude of signal loss, it may be difficult to standardize or quantify test results. Signals with decreasing stability can complicate the interpretation of results. For instance, a fainter line might be misinterpreted as a negative result when it could indicate low levels of the target analyte. Relying on line intensity for quantitative assessment can lead to misleading conclusions, particularly if signal fading is not accounted for. This type of fading is dependent on the dynamic flow of reagents such as in a LFA, but usually not observed in assays such as ELISA, magnetic bead assays or classical dipstick assays.

Therefore, the present invention provides for an improved test for determining microbial contamination in oily liquids such as oils or fuel (including e.g., crude oil), fats or grease. In particular, the present invention provides for an improved LFA method and test kit which reduces or avoids fading of the test line during a period of at least 30 minutes following the test line formation.

According to specific examples, it has been found that an unusual high concentration of Ca⁺⁺ in the reaction buffer in a lateral flow assay with Ca⁺⁺-dependent C-type lectins, when used to detect mannose-containing structures in samples from oily fluids can prevent the rapid fading of the positive signal.

It can be shown that samples of oily fluids induce a fading of the lateral flow assay in a variety of assays with different mannose binding proteins. This rapid fading of signal can be prevented by utilizing an assay buffer with unusual high Ca⁺⁺ concentrations. The stabilized assay signals allow for a high sensitivity detection of indicators for microbial contamination in oily fluids.

It can be concluded that the addition of sufficient amount of Ca⁺⁺ to at least either one of the extraction buffer, the sample buffer, the conjugate buffer or the sample pad buffer may allow to get stable signals and sensitive detection of microbial mannose containing structures from aqueous extracts of oily substances.

The invention is further described by one or more of the following items.

The present invention is specifically described by one or more of the following items:
1. An analytical method to detect microbial contaminants in an oily liquid, comprising:
   a) preparing an aqueous liquid from the oily liquid, thereby obtaining glycan-containing microbial material originating from any microbial contaminants comprised in the oily liquid;
   b) determining the presence of glycans in the aqueous liquid using a lectin-based lateral-flow assay (LFA) that employs mannose-binding protein (MBP) as capture agent of a test region of a lateral flow membrane and as capture agent bound to flowable, identifiable reporter nanoparticles, using a capture reaction buffer (CRB) which comprises 100 to 500 mM free calcium ions, a detergent, a buffer agent and a pH ranging between 6.5 and 9.0, thereby allowing identification of an MBP sandwiched capture product at the test region, which indicates microbial contamination.
2. The method of item 1, wherein the MBP comprises at least one mannose-binding C-type lectin domain (CTLD), preferably wherein said MBP comprises at least 90% sequence identity to any one of SEQ ID NO:1-6, or a CTLD of any of the foregoing.
3. The method of item 1 or 2, wherein the MBP comprises or consists of a CTLD which comprises SEQ ID NO:15.
4. The method of any one of items 1 to 3, wherein said MBP is comprised in an MBP preparation which comprises MBP multimers, wherein 10%-90% of the MBP is comprised as multimers smaller than 140 kDa, preferably wherein the multimers smaller than 140 kDa areMBP dimers and/or trimers.
5. The method of any one of items 1 to 4, wherein said MBP is a mannose-binding lectin (MBL).
6. The method of any one of items 1 to 5, wherein said MBP
   a) comprises at least one mannose-binding C-type lectin domain (CTLD), preferably wherein said MBP comprises at least 90% sequence identity to any one of SEQ ID NO:1-6, or a CTLD of any of the foregoing, or
   b) the MBP comprises or consists of a CTLD which comprises SEQ ID NO:15.
7. The method of any one of items 1 to 6, wherein the same MBP preparation is used both, at the test region and on the reporter nanoparticles.
8. The method of any one of items 1 to 7, wherein the oily liquid is an oil or oily aqueous condensate or emulsion, preferably wherein the oily liquid originates from crude oil, petroleum, mineral oil, or a fraction of any of the foregoing, or a fuel such as kerosene, petrol, diesel, or biodiesel.
9. The method of any one of items 1 to 8, wherein the aqueous liquid is prepared by extracting the oily liquid using an extraction buffer that comprises 100 to 500 mM free calcium ions, a detergent, a buffer agent and a pH ranging between 6.5 and 9.0.
10. The method of any one of items 1 to 9, wherein the CRB is used as running buffer of the LFA.
11. The method of any one of items 1 to 10, wherein a mannan is used at a control region of the lateral flow membrane.
12. The method of any one of items 1 to 11, wherein the microbial contaminants are any one or more of bacteria, yeast, fungi, or spores, preferably originating from *Methylobacterium sp., Burkholderia sp., Rhodococcus erythropolis, Hormoconis resinae, Bacillus pumilus, Yarrowia lipolytica,* or *Candida tropicalis.*
13. An analytical test kit for detecting microbial contamination in an oily liquid, comprising:
   a) a lectin-based lateral-flow assay (LFA) device, which employs mannose-binding protein (MBP) as capture agent that is bound to flowable, identifiable reporter nanoparticles at a reaction zone of a lateral flow membrane; and
   b) a running buffer which comprises a detergent, a buffer agent and a pH ranging between 6.5 and 9.0, and which sets an amount of 100 to 500 mM free calcium ions at the reaction zone.
14. The test kit of item 13, wherein said MBP is comprised in an MBP preparation which comprises MBP multimers, wherein 10%-90% of the MBP is comprised as multimers smaller than 140 kDa, preferably wherein the multimers smaller than 140 kDa are dimers and/or trimers.
15. The test kit of item 12 or 13, wherein said MBP is a mannose-binding lectin (MBL).
16. The test kit of any one of items 13 to 15, wherein said MBP
   a) comprises at least one mannose-binding C-type lectin domain (CTLD), preferably wherein said MBP comprises at least 90% sequence identity to any one of SEQ ID NO:1-6, or a CTLD of any of the foregoing, or
   b) the MBP comprises or consists of a CTLD which comprises SEQ ID NO:15.
17. The test kit of any one of items 13 to 16, wherein the running buffer comprises:
   a) 100 to 500 mM free calcium ions; or
   b) an amount of free calcium ions which complements an amount of free calcium ions that is provided within the LFA device; or
   c) an amount of free calcium ions which complements an amount of free calcium ions that is provided within the sample that is applied to the LFA device and/or an amount of free calcium ions that is provided within the LFA device.
18. The test kit of any one of items 13 to 17, wherein said reaction zone is configured to receive a sample, which reaction zone comprises the MBP bound to the reporter nanoparticles at a conjugate zone, and a detection zone comprising a test region where immobilized MBP is used as a capture agent, and a control region where a positive control is immobilized.
19. The test kit of any one of items 13 to 18, wherein the LFA device comprises one or more of the following features:
   a) the reporter nanoparticles comprise or consist of a tracer, preferably a gold, silver, platinum, carbon, fluorescence, colored latex or magnetic tracer, preferably colloidal gold.
   b) the lateral flow membrane is cellulose-based or synthetic fiber-based, preferably a nitrocellulose membrane or other cellulose-based paper;
   c) the same MBP preparation is used as capture agent that is bound to the reporter nanoparticles and on the test line;
   d) the MBP at the test region is immobilized in or on the lateral flow membrane and resides at a fixed part of the membrane;
   e) mannan is used as positive control, or a microbe-structure independent ligand and ligand-specific target are used as a test control, wherein the ligand is provided as a nanoparticle-conjugate at the conjugate zone and the ligand-specific target is immobilized at the lateral flow membrane.
20. Use of a test kit of any one of items 13 to 19, in a method of any one of items 1 to 12.
21. The method of any one of items 1 to 12, wherein a test kit of any one of items 13 to 19 is used.
22. A method of reducing fading of the intensity of a test signal in a reaction zone of a lateral flow membrane of a lectin-based lateral-flow assay (LFA) device, which assay determines the presence of glycans in an aqueous liquid produced from an oily sample liquid by reacting with a mannose-binding protein (MBP) as capture agent in the reaction zone using a capture reaction buffer (CRB) which comprises 100 to 500 mM free calcium ions, a detergent, a buffer agent and a pH ranging between 6.5 and 9.0.
23. The method of item 22, wherein the assay detects microbial contaminants in an oily liquid according to the method of any one of items 1 to 12, or wherein a test kit of any one of items 13 to 19 is used.
24. The method of item 22 or 23, wherein fading is reduced or avoided within at least 30 minutes following producing the test signal.

In particular, the invention is further described by one or more of the following items.
1. An analytical method to detect microbial contaminants in an oily liquid, comprising:
   a) preparing an aqueous liquid from the oily liquid, thereby obtaining glycan-containing microbial material originating from any microbial contaminants comprised in the oily liquid;
   b) determining the presence of glycans in the aqueous liquid using a lectin-based lateral-flow assay (LFA) that employs mannose-binding protein (MBP) as capture agent of a test region of a lateral flow membrane and as capture agent bound to flowable, identifiable reporter nanoparticles, using a capture reaction buffer (CRB) which comprises 100 to 500 mM free calcium ions, a detergent, a buffer agent and a pH ranging between 6.5 and 9.0, thereby allowing identification of an MBP sandwiched capture product at the test region, which indicates microbial contamination, wherein said MBP is comprised in an MBP preparation which comprises MBP multimers, wherein 10%-90% of the MBP is comprised as multimers smaller than 140 kDa.
2. The method of item 1, wherein the multimers smaller than 140 kDa are MBP dimers and/or trimers.
3. The method of item 1 or 2, wherein said MBP is a mannose-binding lectin (MBL).
4. The method of any one of items 1 to 3, wherein said MBP
   a) comprises at least one mannose-binding C-type lectin domain (CTLD), preferably wherein said MBP comprises at least 90% sequence identity to any one of SEQ ID NO:1-6, or a CTLD of any of the foregoing, or
   b) the MBP comprises or consists of a CTLD which comprises SEQ ID NO:15.
5. The method of any one of items 1 to 4, wherein the same MBP preparation is used both, at the test region and on the reporter nanoparticles.
6. The method of any one of items 1 to 5, wherein the oily liquid is an oil or oily aqueous condensate or emulsion, preferably wherein the oily liquid originates from crude oil, petroleum, mineral oil, or a fraction of any of the foregoing, or a fuel such as kerosene, petrol, diesel, or biodiesel.
7. The method of item 6, wherein the oily liquid is a fuel.
8. The method of any one of items 1 to 7, wherein the aqueous liquid is prepared by extracting the oily liquid using an extraction buffer that comprises 100 to 500 mM free calcium ions, a detergent, a buffer agent and a pH ranging between 6.5 and 9.0.
9. The method of any one of items 1 to 8, wherein the CRB is used as running buffer of the LFA.
10. The method of any one of items 1 to 9, wherein a mannan is used at a control region of the lateral flow membrane.
11. The method of any one of items 1 to 10, wherein the microbial contaminants are any one or more of bacteria, yeast, fungi, or spores, preferably originating from *Methylobacterium sp., Burkholderia sp., Rhodococcus erythropolis, Hormoconis resinae, Bacillus pumilus, Yarrowia lipolytica,* or *Candida tropicalis.*
12. An analytical test kit for detecting microbial contamination in an oily liquid, comprising:
   a) a lectin-based lateral-flow assay (LFA) device, which employs mannose-binding protein (MBP) as capture agent that is bound to flowable, identifiable reporter nanoparticles at a reaction zone of a lateral flow membrane; and
   b) a running buffer which comprises a detergent, a buffer agent and a pH ranging between 6.5 and 9.0, and which sets an amount of 100 to 500 mM free calcium ions at the reaction zone,
   wherein said MBP is comprised in an MBP preparation which comprises MBP multimers, wherein 10%-90% of the MBP is comprised as multimers smaller than 140 kDa.
13. The test kit of item 12, wherein the multimers smaller than 140 kDa are MBP dimers and/or trimers.
14. The test kit of item 12 or 13, wherein said MBP is a mannose-binding lectin (MBL).
15. The test kit of any one of items 12 to 14, wherein said MBP
   a) comprises at least one mannose-binding C-type lectin domain (CTLD), preferably wherein said MBP comprises at least 90% sequence identity to any one of SEQ ID NO:1-6, or a CTLD of any of the foregoing, or
   b) the MBP comprises or consists of a CTLD which comprises SEQ ID NO:15.
16. The test kit of any one of items 12 to 15, wherein the running buffer comprises:
   a) 100 to 500 mM free calcium ions; or
   b) an amount of free calcium ions which complements an amount of free calcium ions that is provided within the LFA device; or
   c) an amount of free calcium ions which complements an amount of free calcium ions that is provided within the sample that is applied to the LFA device and/or an amount of free calcium ions that is provided within the LFA device.
17. The test kit of any one of items 12 to 16, wherein said reaction zone is configured to receive a sample, which reaction zone comprises the MBP bound to the reporter nanoparticles at a conjugate zone, and a detection zone comprising a test region where immobilized MBP is used as a capture agent, and a control region where a positive control is immobilized.
18. The test kit of any one of items 12 to 17, wherein the LFA device comprises one or more of the following features:
   a) the reporter nanoparticles comprise or consist of a tracer, preferably a gold, silver, platinum, carbon, fluorescence, colored latex or magnetic tracer, preferably colloidal gold.
   b) the lateral flow membrane is cellulose-based or synthetic fiber-based, preferably a nitrocellulose membrane or other cellulose-based paper;
   c) the same MBP preparation is used as capture agent that is bound to the reporter nanoparticles and on the test line;
   d) the MBP at the test region is immobilized in or on the lateral flow membrane and resides at a fixed part of the membrane;
   e) mannan is used as positive control, or a microbe-structure independent ligand and ligand-specific target are used as a test control, wherein the ligand is provided as a nanoparticle-conjugate at the conjugate zone and the ligand-specific target is immobilized at the lateral flow membrane.
19. Use of a test kit of any one of items 12 to 18, in a method of any one of items 1 to 11.

### EXAMPLES

### Example 1: Effect of high concentrations of calcium on stability of Test-Signal

### 1.1 Labelling of mannose binding proteins

For preparation of various gold-nanoparticle-mannose binding protein conjugates, BioReady^{™} 40 nm Bare Gold (nanoComposix, USA; Product Number AUCR40) is employed. The recommendations of the manufacturer are followed. In short, a pH titration, according to the manufacturer protocols, to conjugate a mannose binding protein to 250 µL of OD20 BioReady^{™} 40 nm Bare Gold is performed (Colloidal gold concentrations are often expressed in terms of optical density or absorbance at 530 nm with a 1 cm pathlength cuvette).

A pH range at 7, 8, and 9 is evaluated to determine the optimal point for passive binding. Protein can be purified into the appropriate buffer using spin columns or dialysis tubing with the appropriate molecular weight cut-off. The proteins for producing the conjugate are at a concentration 1 mg/mL. For passive adsorption to 40 nm gold, loading 100 µg of protein per mL of OD20 gold can be utilized. For the optimal pH of adsorption, the pH needs to be titrated according to the manufacturer's protocol:
1. Prepare a set of 3 eppi tubes. Label each of the tubes 7A, 8A, and 9A to correspond with each pH point.
2. Add 12.5 µL of each 100 mM buffer to its respectively labelled tube. The volume of buffer added should be around 1/20 of the total working volume of gold.
3. Add 20 µg of mannose binding protein into each of the tubes containing the buffer from step #2.
4. Add 250 µL of OD20 gold to each of the tubes from step #3. Vortex and incubate on the rotator for 10 minutes.
5. Aliquot 50 µL of 10% NaCl into another separate set of 3 eppi tubes. Label each of the tubes 7B, 8B, and 9B.
6. After about 10 minutes of incubation for the "A" tubes (buffer + MBP + gold):
   a. Pipette 50 µL of the conjugate from each "A" tube into each respective "B" tube containing NaCl.
   b. Leave the remaining "A" tube volume to incubate for an additional 20 minutes.
7. Vortex the NaCl + conjugate mixture in the "B" tubes. Incubate on rotator for about 10 minutes and observe any color change:
   a. Stable conjugate: the gold maintains its red color.
   b. Unstable conjugate: the gold becomes purple, gray, clear, or precipitated. Move forward with the pH condition(s) that produced a stable conjugate.
8. After the "A" tubes from step 6b have incubated for a total of 30 minutes, select the tubes that correlated to the stable "B" tubes. Add 1/10 volume of conjugate block buffer so that the final BSA concentration in each "A" tube is around 1%.
9. Incubate for 30 minutes on rotator.
10. Centrifuge the conjugate at 3600 RCF for 3 minutes.
11. Carefully remove the supernatant and resuspend with conjugate diluent buffer to the target OD (e.g. 200 µL final volume for OD20). Vortex and bath sonicate if needed to fully resuspend the conjugate.
12. Perform UV-Vis reading and check for the final OD.
13. Store conjugate at 4°C until use. Do not freeze.

**Table 1**

| Protein source | SEQ ID | Theoretical pl |
|---|---|---|
| Recombinant Human MBL Protein, CF (R&D Systems Catalog #: 9086-MB) | SEQ ID NO:1 rhMBL | 5.23 |
| Mouse MBL-1 R&D Systems Catalog no.: 2077-MB | SEQ ID NO:2 rmMBL-1 | 7.55 |
| Recombinant Mouse MBL-2 Protein R&D Systems Catalog #: 2208-MB | SEQ ID NO:3 rmMBL-2 | 4.94 |
| Recombinant Bovine Mannose-binding protein C(MBL), His-tagged, CUSABIO Technology (CSB-BP013540BO) | SEQ ID NO:8 His-rbMBL | 6.12 |
| Recombinant human MBL/His Tag, The Antibody Lab GmbH, Vienna Austria | SEQ ID NO:9 rhMBL-His | 6.00 |
| Recombinant Human DC-SIGN/CD209 Protein R&D Systems Catalog # 9136-DC | SEQ ID NO:5 rhCD209 | 5.23 |
| Recombinant human MBL-domain/His, The Antibody Lab GmbH | SEQ ID NO:7 rhMBL-domain-His | 5.61 |

### 1.2 Setting up the lateral flow assay:

The assay components can be screened by use of the Lateral Flow Material Starter Kit (nanoComposix, USA; Product number: MSKR). The Kit comes with a variety of components, respectively, such as membranes, wick pad, conjugate pad, and sample pad. The following materials have been tested according to the manufacturer's protocols:
Membranes: UniSart^{®} CN 95 from Sartorius, Vivid^{™} 120 from Pall, MDI 70, MDI 90 and MDI 150 from MDI; Wickpad Grad 222 from Ahlstrom; sample pad 1281 cotton, from Ahlstrom; conjugate pads 8950 glass fiber, 8951 glass fiber, 6613 polyester fiber, 6614 polyester fiber, all from Ahlstrom. The optimization was performed according to the manufacturer's instruction. A signal to noise ratio is selected for by utilizing Yeast Mannan (Sigma M7504) as a positive sample.

### 1.3 Lateral Flow Test Setup with recombinant human MBP:

### 1.3.1 Gold conjugate production

In a 2 ml Eppendorf tube, MBL (R&D Systems, USA, catalog number 9086-MB) was diluted to 4 microgram/ml in 1,5 ml conjugate buffer (25 mM borate, pH 7.0). After addition of 150 microliters of gold nanoparticles (40 nm gold nanoparticles, OD10, from Abcam, England; catalog number ab269930) the Eppendorf was inverted immediately several times to ensure homogenous distribution. The mix was incubated for 15 min at room temperature on a rotation mixer. Subsequently, 75 microliters of blocking buffer (2mM borate, 10% BSA, 0.1% Tween 20, 0.1% sodium azide, pH9.0) was added, and the tube was again incubated for 15 minutes at room temperature on a rotation mixer. The mix was then centrifuged for 10 minutes at 2500 x g, the supernatant was carefully removed and the pellet is resuspended in buffer (2mM borate, 1% BSA, 0.1% Tween 20, 0.1% sodium azide, pH9.0) by gently flicking the tube.

### 1.3.2 Quality control and quantification

The absorbance of the resuspended conjugate was measured at 530 nm, 550 nm and 600 nm, utilizing a Tecan SPARK 10 spectrophotometer, the cuvette had a path length of 1 cm. The OD of pure resuspension buffer was subtracted from the respective sample value. The value of the measurement at 530 nm was defined as GU/microliter. The aggregation factor is determined by the ratio OD550/OD600. This factor is to be below 3.5 in order to pass the quality control of the MBP gold nanoparticle conjugate.

The test is performed by the half strip spotting method in which MBP is spotted as a drop on the LFA strip.:
Nitrocellulose strips (CN95; Sartorius) are spotted at the test zone with 5 microliter mannose binding protein (0.1 mg/ml in 5mM Borate; 150 mM NaCl; 0,0625% Tween-20; pH 8); strips are dried for 30 minutes at 37 degrees Celsius; an amount of OD15 for gold-mannose binding protein-conjugate per strip on conjugate pad (conjugate resuspension buffer is 2mM borate, 0.1% Tween-20, 0.1% azide, 1% BSA, pH 9). The strips are dried for 1 hour at 37°C and used immediately after drying or stored at room temperature in a desiccated bag. 500 microliters of samples diluted in capture reaction buffer are prepared. The sample is a 1:5 dilution in capture reaction buffer of either mannan in sample buffer (50 ng/ml; sample buffer is 12.5 mM Borate-buffer, pH 8.0) or in aqueous extract of Jet A1 Fuel (i.e. fuel spiked with 100 ng mannan (Sigma M7504)/ml). The spiked fuel is extracted with sample buffer in a ratio of 1:150 aqueous buffer/fuel by thoroughly shaking for 30 seconds. After gravity separation of the phases (approx. 5 minutes standing on the lab table), 100 microliters of aqueous phase are added to the test. The capture reaction buffer is 15 mM Tris pH 8 with 0.3% Tween-20. The variations of the capture reaction buffer include either 12,5 mM EDTA, 12,5 mM CaCl₂, 125 mM CaCl₂ and 625 mM CaCl2, respectively. 100 microliter of each sample is applied to the sample pad of a LFA strip and the development of the color at the spot zone is observed. The reaction is observed visually and rated from zero (-) to weakly reddish (+) to red (++) to strong and deep red (+++). The signal in the test zone is formed after approximately 2 min. The signal is again checked and recorded regularly for 30 minutes. Table 2 summarizes the results after 4 minutes and 30 minutes after starting the assays, for an assay utilizing Recombinant Human MBL Protein (R&D Systems Catalog #: 9086-MB) both coated on the test zone as well as a gold conjugate.

**Table 2:**

| Mannan in | Capture Reaction Buffer with | Signal after 4 minutes | Signal after 30 minutes |
|---|---|---|---|
| Sample buffer | 10 mM EDTA | - | - |
| Sample Buffer | 10mM CaCl2 | +++ | +++ |
| Sample Buffer | 100mM CaCl2 | +++ | +++ |
| Sample buffer | 500 mM CaCl2 | +++ | +++ |
| Fuel | 10 mM EDTA | - | - |
| Fuel | 10mM CaCl2 | ++ | + |
| Fuel | 100mM CaCl2 | +++ | ++ |
| Fuel | 500 mM CaCl2 | +++ | +++ |

It can be seen from results shown in Table 2 that addition of unusually high concentrations of calcium stabilizes the signal derived from fuel extract samples. Changing to other mannose binding proteins for coating and/or for conjugates as well as changing the pH of the capture reaction buffer between 6.5 and 9 did not significantly change the results in Table 2.

MBL is an exemplary MBP as described herein. There are other mannose-binding proteins such as DC-SIGN (e.g., R&D Systems, USA, catalog number 9136-DC), Dectin-2 (e.g., R&D Systems, USA, catalog number 3114-DC), MMR (e.g., R&D Systems, USA, catalog number 2534-MR) or Langerin (e.g., R&D Systems, USA, catalog number 2088-LN), for which the same dependence of Ca⁺⁺-ion concentration is determined for signal stability in a lateral flow assay setup as described herein.

The signal fading effect at low calcium-ion concentrations has been determined in an LFA assay using MBP (or MBL) as capture agent, but is typically not observed in an analogous sandwich ELISA format of test.

### Example 2: Detection of microbial contamination in fuel samples

The assay as described in Example 1 was used to detect microbes or microbial matter in fuel. Samples for the LFA were either watery samples from fuel tanks or kerosene. The samples were treated as in the example before: Extraction by diluting and mixing the sample with sample buffer (1:2) by vortexing, then a centrifugation at room temperature was performed to separate the phases, 10 microliters of the aqueous phase are then transferred to 40 microliter of capture reaction buffer with 10 and 500 mM CaCl₂ respectively ("LFA 10mM Ca" and "LFA 500mM Ca"), mixed and applied to the sample pad of the lateral flow strip. The same samples were tested with a standard method for determination of the viable aerobic microbial content (bacteria and fungi respectively; "IP385 bacteria" and IP385 fungi") of middle distillate fuels and associated water (IP385, standard protocol available at the ASTM manual, "IP 385-99 Determination of the Viable Aerobic Microbial Content of Fuels and Fuel Components Boiling Below 390 °C-Filtration and Culture Method" (ASTM International (ASTM), USA, June 2003),.

The results in Table 3 show that it is possible to detect with the mannose binding protein lateral flow assay with high calcium concentration in the reaction buffer the equivalent of a low number of cfu/ml in the samples.

**Table 3**

| Sample | IP385 bacteria | IP385 fungi | LFA 10mM Ca | LFA 500mM Ca | |
|---|---|---|---|---|---|
| 1 | 71 | 350 | + | +++ | |
| 2 | 13 | bdl | - | +++ | |
| 3 | 9 | bdl | - | ++ | |
| 4 | 7 | 300 | + | +++ | |

| | | | | | |
|---|---|---|---|---|---|
| IP385 numbers are cfu/ml; bdl...below detection limit | | | | | |

### Example 3: Detection of indicator strains

The following indicator strains of bacteria and fungi can be grown on the IP385 media, a sample of a colony of each of the strains is suspended in sample buffer and then mixed (1:5) with capture reaction buffer with 500 mM CaCl2. After centrifugation, 50 microliters of the clear supernatant are applied to a lateral flow strip as described in Example 1. It can be shown that Rhodococcus erythropolis, a Burkholderia sp. mix, a Methylobacterium sp., Bacillus pumilus, Yarrowia lipolytica, Candida tropicalis and Hormoconis resinae react positive.

### Example 4: Expression of recombinant human mannose binding protein in CHO

### 4.1. Cloning:

Protein sequence source: Uniprot P11226
Expression vector: VB230228 Sleeping Beauty Gene Expression Vector System (Vector Builder)
Host Cell strain: CHO-K1 (The Antibody Lab, Austria)
Expressed recombinant human MBP protein sequence with N-terminal albumin signal peptide (*italic,* cleaved during secretion), C-terminal 6xHis tag **(bold)** and extra 2 amino acids (underlined) (cloning specific; 27 kDa), SEQ ID NO:16:

MKWVTFISLLFLFSSAYSSLFPSLPLLLLSMVAASYSETVTCEDAQKTCPAVIACSSPGINGFP
GKDGRDGTKGEKGEPGQGLRGLQGPPGKLGPPGNPGPSGSPGPKGQKGDPGKSPDGDSSLAASE
RKALQTEMARIKKWLTFSLGKQVGNKFFLTNGEIMTFEKVKALCVKFQASVATPRNAAENGAIQ
NLIKEEAFLGITDEKTEGQFVDLTGNRLTYTNWNEGEPNNAGSDEDCVLLLKNGQWNDVPCSTS
HLAVCEFPILEHHHHHH

The gene cassette including Cricetulus griseus-optimized hMBP DNA with N-terminal albumin leader sequence was cloned into Sleeping Beauty cloning vector together with the Neomycin resistance gene cassette under the control of PGK promoter. CHO-K1 cells were transfected with the cloning vector and the Sleeping Beauty helper vector encoding the transposase, which cuts the IR/DR sequences and inserts the cassette into the cell genome. Cells were subjected to harsh antibiotic selection and the remaining live cells were processed by limiting dilution for single clone expansion. Limiting dilution and single clone expansion yielded 28 cell clones which were genotyped for presence of recombinant MBP gene. Two detected positive clones were expanded for cell bank storage and tested for expression yield in fed batch production.

### 4.2. Protein Production:

### Culture conditions:

Cells are grown in CD CHO Medium (Gibco, 10743-029)/L-glutamine/Anti-clumping agent (Gibco, 01-0057D6) at 37 °C, 5 % CO2, shaking in a rack at min 160 rpm at an angle of 45° and passaged every 3-4 days at 1E5/ml.

### Fed batch production:

For protein production cells were seeded at 3E5/ml in HyClone ActiPro medium (GE,SH31039.02)/ ultra-glutamine/ anti-clumping agent in shaker flasks and incubated for 10 days at 37 °C, 5 % CO₂, shaking at 160 rpm. On days 3, 6, 7 and 8 cultures were fed with 4% HyClone Cell Boost 7A (GE, 15805261). Cultures were harvested by centrifugation 20 min at 4000 rpm and sterile filtered. Production supernatants were analysed for MBP expression by SDS-PAGE.

### 4.3. Purification

Binding/wash buffer: 10 mM Tris pH 7.5/ 150 mM NaCl/5 mM imidazole.
Elution buffer: 10 mM Tris pH 7.5/0.5 M NaCl/ 0.5 M imidazole.
Dilute 20 ml cell culture supernatant 1:1 with 10 mM Tris/150 mM NaCl/5 mM imidazole pH 7.5.
Pipet 1 ml of Ni-NTA agarose beads (Qiagen, ID 30210) into a fresh 15 ml centrifuge tube (adjust the volume of beads for more than 4 ml supernatant).
Spin down 2 min at 2000 rcf, resuspended the beads in 2 ml binding buffer and spin down again.
Resuspend the beads in 20 ml supernatant and incubate the beads at 4°C for 1 h with end-to-end rotation.
Load the slurry on the gravity flow column and collect the flow through for control.
Wash 2x with 2 ml binding buffer (keep the flow through for control).
Add 4x 0.5 ml elution buffer and collect elutions E1 - E4.
Store samples at 4°C.
Check the quality of purified samples on PAGE.
Pool elution fractions with visible protein bands of expected size and concentrate/exchange buffer for PBS using Vivaspin centrifugal devices with 10 K MW cut off.

## Claims

1. An analytical method to detect microbial contaminants in an oily liquid, comprising:
a) preparing an aqueous liquid from the oily liquid, thereby obtaining glycan-containing microbial material originating from any microbial contaminants comprised in the oily liquid;
b) determining the presence of glycans in the aqueous liquid using a lectin-based lateral-flow assay (LFA) that employs mannose-binding protein (MBP) as capture agent of a test region of a lateral flow membrane and as capture agent bound to flowable, identifiable reporter nanoparticles, using a capture reaction buffer (CRB) which comprises 100 to 500 mM free calcium ions, a detergent, a buffer agent and a pH ranging between 6.5 and 9.0, thereby allowing identification of an MBP sandwiched capture product at the test region, which indicates microbial contamination.

2. The method of claim 1, wherein the MBP comprises at least one mannose-binding C-type lectin domain (CTLD), preferably wherein said MBP comprises at least 90% sequence identity to any one of SEQ ID NO:1-6, or a CTLD of any of the foregoing.

3. The method of claim 1 or 2, wherein the MBP comprises or consists of a CTLD which comprises SEQ ID NO:15.

4. The method of any one of claims 1 to 3, wherein said MBP is a mannose-binding lectin (MBL).

5. The method of any one of claims 1 to 4, wherein said MBP
a) comprises at least one mannose-binding C-type lectin domain (CTLD), preferably wherein said MBP comprises at least 90% sequence identity to any one of SEQ ID NO:1-6, or a CTLD of any of the foregoing, or
b) the MBP comprises or consists of a CTLD which comprises SEQ ID NO:15.

6. The method of any one of claims 1 to 5, wherein the same MBP preparation is used both, at the test region and on the reporter nanoparticles.

7. The method of any one of claims 1 to 6, wherein the oily liquid is an oil or oily aqueous condensate or emulsion, preferably wherein the oily liquid originates from crude oil, petroleum, mineral oil, or a fraction of any of the foregoing, or a fuel such as kerosene, petrol, diesel, or biodiesel.

8. The method of any one of claims 1 to 7, wherein the aqueous liquid is prepared by extracting the oily liquid using an extraction buffer that comprises 100 to 500 mM free calcium ions, a detergent, a buffer agent and a pH ranging between 6.5 and 9.0.

9. The method of any one of claims 1 to 8, wherein the CRB is used as running buffer of the LFA.

10. The method of any one of claims 1 to 9, wherein a mannan is used at a control region of the lateral flow membrane.

11. The method of any one of claims 1 to 10, wherein the microbial contaminants are any one or more of bacteria, yeast, fungi, or spores, preferably originating from *Methylobacterium sp., Burkholderia sp., Rhodococcus erythropolis, Hormoconis resinae, Bacillus pumilus, Yarrowia lipolytica,* or *Candida tropicalis.*

12. An analytical test kit for detecting microbial contamination in an oily liquid, comprising:
a) a lectin-based lateral-flow assay (LFA) device, which employs mannose-binding protein (MBP) as capture agent that is bound to flowable, identifiable reporter nanoparticles at a reaction zone of a lateral flow membrane; and
b) a running buffer which comprises a detergent, a buffer agent and a pH ranging between 6.5 and 9.0, which running buffer comprises 100 to 500 mM free calcium ions and sets an amount of 100 to 500 mM free calcium ions at the reaction zone,
wherein the reaction zone comprises the MBP bound to the reporter nanoparticles at a conjugate zone, and a test region comprising a test line where immobilized MBP is used as a capture agent.

13. The test kit of claim 12, wherein said MBP is comprised in an MBP preparation which comprises MBP multimers, wherein 10%-90% of the MBP is comprised as multimers smaller than 140 kDa, preferably wherein the multimers smaller than 140 kDa are dimers and/or trimers.

14. The test kit of claim 12 or 13, wherein said MBP is a mannose-binding lectin (MBL).

15. The test kit of any one of claims 12 to 14, wherein said MBP
a) comprises at least one mannose-binding C-type lectin domain (CTLD), preferably wherein said MBP comprises at least 90% sequence identity to any one of SEQ ID NO:1-6, or a CTLD of any of the foregoing, or
b) the MBP comprises or consists of a CTLD which comprises SEQ ID NO:15.

16. The test kit of any one of claims 12 to 15, wherein:
a) the running buffer comprises one or more different detergents selected from the group consisting of NP-40, Triton-X100, Tween 20, Tween-80, CHAPS, CA-630, Brij-35, Triton-X114, GDN, GLC and Pluronic-F68; and
b) the running buffer comprises one or more different buffer substances selected from the group consisting of Phosphate, Borate, MES, HEPES or Tris.

17. The test kit of any one of claims 12 to 16, wherein said reaction zone is configured to receive a sample, which reaction zone comprises the MBP bound to the reporter nanoparticles at the conjugate zone, and a detection zone comprising the test region where immobilized MBP is used as a capture agent, and a control region where a positive control is immobilized.

18. The test kit of any one of claims 12 to 17, wherein the LFA device comprises one or more of the following features:
a) the reporter nanoparticles comprise or consist of a tracer, preferably a gold, silver, platinum, carbon, fluorescence, colored latex or magnetic tracer, preferably colloidal gold.
b) the lateral flow membrane is cellulose-based or synthetic fiber-based, preferably a nitrocellulose membrane or other cellulose-based paper;
c) the same MBP preparation is used as capture agent that is bound to the reporter nanoparticles and on the test line;
d) the MBP at the test region is immobilized in or on the lateral flow membrane and resides at a fixed part of the membrane;
e) mannan is used as positive control, or a microbe-structure independent ligand and ligand-specific target are used as a test control, wherein the ligand is provided as a nanoparticle-conjugate at the conjugate zone and the ligand-specific target is immobilized at the lateral flow membrane.

19. Use of a test kit of any one of claims 12 to 18, in a method of any one of claims 1 to 12.

20. A method of reducing fading of the intensity of a test signal in a reaction zone of a lateral flow membrane of a lectin-based lateral-flow assay (LFA) device, which assay determines the presence of glycans in an aqueous liquid produced from an oily sample liquid by reacting with a mannose-binding protein (MBP) as capture agent in the reaction zone using a capture reaction buffer (CRB) which comprises 100 to 500 mM free calcium ions, a detergent, a buffer agent and a pH ranging between 6.5 and 9.0.

21. The method of claim 20, wherein the assay detects microbial contaminants in an oily liquid according to the method of any one of claims 1 to 12, or wherein a test kit of any one of claims 13 to 19 is used.

22. The method of claim 20 or 21, wherein fading is reduced or avoided within at least 30 minutes following producing the test signal.
